# EUROPEAN PATENT SPECIFICATION

(11) **EP 0 987 275 B1**
(45) Date of publication and mention of the grant of the patent: **02.01.2008**
(21) Application number: 99250432.4
(22) Date of filing: 10.09.1996
(51) Int. Cl.: C07K 1/04, A61K 47/48, C07K 7/08, A61K 49/00, A61K 51/08

(54) **Molecules that home to a selected organ or tissue in vivo**
Moleküle, die sich in ausgewählten Organen oder Geweben in-vivo einfinden
Molécules qui s'adressent in vivo vers un organe ou tissu prélevé

(30) Priority: 11.09.1995 US 526708; 11.09.1995 US 526710
(43) Date of publication of application: 22.03.2000
(62) Divisional of application: 96250195.3
(73) Proprietor: LA JOLLA CANCER RESEARCH FOUNDATION, La Jolla, CA 92037 (US)
(72) Inventor: Ruoslahti, Erkki I., Rancho Santa Fe, CA 92067 (US); Pasqualini, Renata, Solana Beach, CA 92075 (US)
(74) Representative: UEXKÜLL & STOLBERG

(56) References cited:
- WO-A-95/14714
- KOIVUNEN E ET AL: "PHAGE LIBRARIES DISPLAYING CYCLIC PEPTIDES WITH DIFFERENT RING SIZES: LIGAND SPECIFICITIES OF THE RGD-DIRECTED INTEGRINS" BIO/TECHNOLOGY, NATURE PUBLISHING CO. NEW YORK, US, vol. 13, no. 3, March 1995 (1995-03), pages 265-270, XP002055920 ISSN: 0733-222X
- ERKKI KOIVUNEN ET AL: "SELECTION OF PEPTIDES BINDING TO THE X5B1 INTEGRIN FROM PHAGE DISPLAY LIBRARY*" JOURNAL OF BIOLOGICAL CHEMISTRY, AMERICAN SOCIETY OF BIOLOCHEMICAL BIOLOGISTS, BIRMINGHAM,, US, vol. 268, no. 27, 25 September 1993 (1993-09-25), pages 20205-20210, XP000396659 ISSN: 0021-9258

### SEQUENCE LISTING,

<110> La Jolla Cancer Research Foundation
<120> Molecules that home to a selected organ or tissue in vivo and methods of identifying same
<130> P052211
<140>
   <141>
<150> US 08/526,708
   <151> 1995-09-11
<150> US 08/526, 710
   <151> 1995-09-11
<160> 59
<170> PatentIn Ver. 2.1
<210> 1
   <211> 9
   <212> PRT
   <213> Artificial Sequence
<220>
   <223> Description of Artificial Sequence: Peptide
<400> 1
   Cys Asn Ser Arg Leu His Leu Arg Cys
   1 5
<210> 2
   <211> 9
   <212> PRT
   <213> Artificial Sequence
<220>
   <223> Description of Artificial Sequence: Peptide
<400> 2
   Cys Glu Asn Trp Trp Gly Asp Val Cys
   1 5
<210> 3
   <211> 9
   <212> PRT
   <213> Artificial Sequence
<220>
   <223> Description of Artificial Sequence: Peptide
<400> 3
   Cys Leu Ser Ser Arg Leu Asp Ala Cys
   1 5
<210> 4
   <211> 8
   <212> PRT
   <213> Artificial Sequence
<220>
   <223> Description of Artificial Sequence: Peptide
<400> 4
   Cys Val Leu Arg Gly Gly Arg Cys
   1 5
<210> 5
   <211> 9
   <212> PRT
   <213> Artificial Sequence
<220>
   <223> Description of Artificial Sequence: Peptide
<400> 5
   Cys Asn Ser Arg Leu Gln Leu Arg Cys
   1 5
<210> 6
   <211> 7
   <212> PRT
   <213> Artificial Sequence
<220>
   <223> Description of Artificial Sequence: Peptide
<400> 6
   Cys Gly Val Arg Leu Gly Cys
   1 5
<210> 7
   <211> 8
   <212> PRT
   <213> Artificial Sequence
<220>
   <223> Description of Artificial Sequence: Peptide
<400> 7
   Cys Lys Asp Trp Gly Arg Ile Cys
   1 5
<210> 8
   <211> 8
   <212> PRT
   <213> Artificial Sequence
<220>
   <223> Description of Artificial Sequence: Peptide
<400> 8
   Cys Leu Asp Trp Gly Arg Ile Cys
   1 5
<210> 9
   <211> 8
   <212> PRT
   <213> Artificial Sequence
<220>
   <223> Description of Artificial Sequence: Peptide
<400> 9
   Cys Thr Arg Ile Thr Glu Ser Cys
   1 5
<210> 10
   <211> 7
   <212> PRT
   <213> Artificial Sequence
<220>
   <223> Description of Artificial Sequence: Peptide
<400> 10
   Cys Glu Thr Leu Pro Ala Cys
   1 5
<210> 11
   <211> 8
   <212> PRT
   <213> Artificial Sequence
<220>
   <223> Description of Artificial Sequence: Peptide
<400> 11
   Cys Arg Thr Gly Thr Leu Phe Cys
   1 5
<210> 12
   <211> 8
   <212> PRT
   <213> Artificial Sequence
<220>
   <223> Description of Artificial Sequence: Peptide
<400> 12
   Cys Gly Arg Ser Leu Asp Ala Cys
   1 5
<210> 13
   <211> 9
   <212> PRT
   <213> Artificial Sequence
<220>
   <223> Description of Artificial Sequence: Peptide
<400> 13
   Cys Arg His Trp Phe Asp Val Val Cys
   1 5
<210> 14
   <211> 8
   <212> PRT
   <213> Artificial Sequence
<220>
   <223> Description of Artificial Sequence: Peptide
<400> 14
   Cys Ala Asn Ala Gln Ser His Cys
   1 5
<210> 15
   <211> 8
   <212> PRT
   <213> Artificial Sequence
<220>
   <223> Description of Artificial Sequence: Peptide
<400> 15
   Cys Gly Asn Pro Ser Tyr Arg Cys
   1 5
<210> 16
   <211> 21
   <212> PRT
   <213> Artificial Sequence
<220>
   <223> Description of Artificial Sequence: Peptide
<400> 16
   Trp Arg Cys Val Leu Arg Glu Gly Pro Ala Gly Gly Cys Ala Trp Phe
   1 5 10 15
   Asn Arg His Arg Leu
   20
<210> 17
   <211> 20
   <212> PRT
   <213> Artificial Sequence
<220>
   <223> Description of Artificial Sequence: Peptide
<400> 17
   Tyr Pro Cys Gly Gly Glu Ala Val Ala Gly Val Ser Ser Val Arg Thr
   1               5                   10                  15
   Met Cys Ser Glu
               20
<210> 18
   <211> 20
   <212> PRT
   <213> Artificial Sequence
<220>
   <223> Description of Artificial Sequence: Peptide
<400> 18
   Leu Asn Cys Asp Tyr Gln Gly Thr Asn Pro Ala Thr Ser Val Ser Val
   1               5                   10                  15
   Pro Cys Thr Val
               20
<210> 19
   <211> 7
   <212> PRT
   <213> Artificial Sequence
<220>
   <223> Description of Artificial Sequence: Peptide
<400> 19
   Leu Ser Ser Arg Leu Asp Ala
   1               5
<210> 20
   <211> 9
   <212> PRT
   <213> Artificial Sequence
<220>
   <223> Description of Artificial Sequence: Peptide
<400> 20
   Val Leu Arg Glu Gly Pro Ala Gly Gly
   1               5
<210> 21
   <211> 7
   <212> PRT
   <213> Artificial Sequence
<220>
   <223> Description of Artificial Sequence: Peptide
<400> 21
   Cys Leu Pro Val Ala Ser Cys
   1 5
<210> 22
   <211> 7
   <212> PRT
   <213> Artificial Sequence
<220>
   <223> Description of Artificial Sequence: Peptide
<400> 22
   Cys Gly Ala Arg Glu Met Cys
   1 5
<210> 23
   <211> 8
   <212> PRT
   <213> Artificial Sequence
<220>
   <223> Description of Artificial Sequence: Peptide
<400> 23
   Cys Lys Gly Arg Ser Ser Ala Cys
   1 5
<210> 24
   <211> 8
   <212> PRT
   <213> Artificial Sequence
<220>
   <223> Description of Artificial Sequence: Peptide
<400> 24
   Cys Trp Ala Arg Ala Gln Gly Cys
   1 5
<210> 25
   <211> 8
   <212> PRT
   <213> Artificial Sequence
<220>
   <223> Description of Artificial Sequence: Peptide
<400> 25
   Cys Leu Gly Arg Ser Ser Val Cys
   1 5
<210> 26
   <211> 8
   <212> PRT
   <213> Artificial Sequence
<220>
   <223> Description of Artificial Sequence: Peptide
<400> 26
   Cys Thr Ser Pro Gly Gly Ser Cys
   1 5
<210> 27
   <211> 8
   <212> PRT
   <213> Artificial Sequence
<220>
   <223> Description of Artificial Sequence: Peptide
<400> 27
   Cys Met Gly Arg Trp Arg Leu Cys
   1 5
<210> 28
   <211> 8
   <212> PRT
   <213> Artificial Sequence
<220>
   <223> Description of Artificial Sequence: Peptide
<400> 28
   Cys Val Gly Glu Cys Gly Gly Cys
   1 5
<210> 29
   <211> 7
   <212> PRT
   <213> Artificial Sequence
<220>
   <223> Description of Artificial Sequence: Peptide
<400> 29
   Cys Val Ala Trp Leu Asn Cys
   1 5
<210> 30
   <211> 7
   <212> PRT
   <213> Artificial Sequence
<220>
   <223> Description of Artificial Sequence: Peptide
<400> 30
   Cys Arg Arg Phe Gln Asp Cys
   1 5
<210> 31
   <211> 7
   <212> PRT
   <213> Artificial Sequence
<220>
   <223> Description of Artificial Sequence: Peptide
<400> 31
   Cys Leu Met Gly Val His Cys
   1 5
<210> 32
   <211> 8
   <212> PRT
   <213> Artificial Sequence
<220>
   <223> Description of Artificial Sequence: Peptide
<400> 32
   Cys Lys Leu Leu Ser Gly Val Cys
   1 5
<210> 33
   <211> 8
   <212> PRT
   <213> Artificial Sequence
<220>
   <223> Description of Artificial Sequence: Peptide
<400> 33
   Cys Phe Val Gly His Asp Leu Cys
   1 5
<210> 34
   <211> 7
   <212> PRT
   <213> Artificial Sequence
<220>
   <223> Description of Artificial Sequence: Peptide
<400> 34
   Cys Arg Cys Leu Asn Val Cys
   1 5
<210> 35
   <211> 7
   <212> PRT
   <213> Artificial Sequence
<220>
   <223> Description of Artificial Sequence: Peptide
<400> 35
   Cys Lys Leu Met Gly Glu Cys
   1 5
<210> 36
   <211> 7
   <212> PRT
   <213> Artificial Sequence
<220>
   <223> Description of Artificial Sequence: Peptide
<400> 36
   Cys Xaa Xaa Xaa Xaa Xaa Cys
   1 5
<210> 37
   <211> 8
   <212> PRT
   <213> Artificial Sequence
<220>
   <223> Description of Artificial Sequence: Peptide
<400> 37
   Cys Xaa Xaa Xaa Xaa Xaa Xaa Cys
   1 5
<210> 38
   <211> 9
   <212> PRT
   <213> Artificial Sequence
<220>
   <223> Description of Artificial Sequence: Peptide
<400> 38
   Cys Xaa Xaa Xaa Xaa Xaa Xaa Xaa Cys
   1 5
<210> 39
   <211> 10
   <212> PRT
   <213> Artificial Sequence
<220>
   <223> Description of Artificial Sequence: Peptide
<400> 39
   Cys Xaa Xaa Xaa Xaa Xaa Xaa Xaa Xaa Xaa
   1 5 10
<210> 40
   <211> 20
   <212> PRT
   <213> Artificial Sequence
<220>
   <223> Description of Artificial Sequence: Peptide
<400> 40
   Xaa Xaa Cys Xaa Xaa Xaa Xaa Xaa Xaa Xaa Xaa Xaa Xaa Xaa Xaa Xaa
   1 5 10 15
   Xaa Cys Xaa Xaa
   20
<210> 41
   <211> 21
   <212> PRT
   <213> Artificial Sequence
<220>
   <223> Description of Artificial Sequence: Peptide
<400> 41
   Xaa Xaa Cys Xaa Xaa Xaa Xaa Xaa Xaa Xaa Xaa Xaa Xaa Xaa Xaa Xaa
   1 5 10 15
   Xaa Xaa Xaa Xaa Xaa
   20
<210> 42
   <211> 21
   <212> DNA
   <213> Artificial Sequence
<220>
   <223> Description of Artificial Sequence: Oligonucleotide
<400> 42
   tgtnnknnkn nknnknnktg t 21
<210> 43
   <211> 9
   <212> PRT
   <213> Artificial Sequence
<220>
   <223> Description of Artificial Sequence: Peptide
<400> 43
   Cys Val Arg Leu Asn Ser Leu Ala Cys
   1 5
<210> 44
   <211> 4
   <212> PRT
   <213> Artificial Sequence
<220>
   <223> Description of Artificial Sequence: Peptide
<400> 44
   Asp Xaa Xaa Arg
   1
<210> 45
   <211> 4
   <212> PRT
   <213> Artificial Sequence
<220>
   <223> Description of Artificial Sequence: Peptide
<220>
   <223> Xaa at position 1 is about 1 to about 10 independently selected amino acids
<220>
   <223> Xaa at position 4 is about 1 to about 10 independently selected amino acids
<400> 45
   Xaa Ser Arg Xaa
   1
<210> 46
   <211> 5
   <212> PRT
   <213> Artificial Sequence
<220>
   <223> Description of Artificial Sequence: Peptide
<220>
   <223> Xaa at position 1 is absent or is about 1 to about 10 independently selected amino acids
<220>
   <223> Xaa at position 5 is about 1 to about 20 independently selected amino acids
<400> 46
   Xaa Val Leu Arg Xaa
   1 5
<210> 47
   <211> 12
   <212> PRT
   <213> Artificial Sequence
<220>
   <223> Description of Artificial Sequence: Peptide
<400> 47
   Cys Xaa Xaa Cys Xaa Xaa Xaa Cys Xaa Xaa Xaa Cys
   1 5 10
<210> 48
   <211> 13
   <212> PRT
   <213> Artificial Sequence
<220>
   <223> Description of Artificial Sequence: Peptide
<400> 48
   Cys Gly Arg Glu Cys Pro Arg Leu Cys Gln Ser Ser Cys
   1 5 10
<210> 49
   <211> 13
   <212> PRT
   <213> Artificial Sequence
<220>
   <223> Description of Artificial Sequence: Peptide
<400> 49
   Cys Gly Glu Ala Cys Gly Gly Gln Cys Ala Leu Pro Cys
   1 5 10
<210> 50
   <211> 13
   <212> PRT
   <213> Artificial Sequence
<220>
   <223> Description of Artificial Sequence: Peptide
<400> 50
   Cys Asn Gly Arg Cys Val Ser Gly Cys Ala Gly Arg Cys
   1 5 10
<210> 51
   <211> 13
   <212> PRT
   <213> Artificial Sequence
<220>
   <223> Description of Artificial Sequence: Peptide
<400> 51
   Cys Gly Leu Met Cys Gln Gly Ala Cys Phe Asp Val Cys
   1 5 10
<210> 52
   <211> 6
   <212> PRT
   <213> Artificial Sequence
<220>
   <223> Description of Artificial Sequence: Peptide
<400> 52
   Trp Gly Thr Gly Leu Cys
   1 5
<210> 53
   <211> 7
   <212> PRT
   <213> Artificial Sequence
<220>
   <223> Description of Artificial Sequence: Peptide
<400> 53
   Cys Ser Val Ala Asn Ser Cys
   1 5
<210> 54
   <211> 7
   <212> PRT
   <213> Artificial Sequence
<220>
   <223> Description of Artificial Sequence: Peptide
<400> 54
   Cys Ser Ser Thr Met Arg Cys
   1 5
<210> 55
   <211> 8
   <212> PRT
   <213> Artificial Sequence
<220>
   <223> Description of Artificial Sequence: Peptide
<400> 55
   Cys Leu Ser Gly Ser Leu Ser Cys
   1 5
<210> 56
   <211> 9
   <212> PRT
   <213> Artificial Sequence
<220>
   <223> Description of Artificial Sequence: Peptide
<400> 56
   Cys Ile Glu Gly Val Leu Gly Gly Cys
   1 5
<210> 57
   <211> 9
   <212> PRT
   <213> Artificial Sequence
<220>
   <223> Description of Artificial Sequence: Peptide
<400> 57
   Cys Asp Cys Arg Gly Asp Cys Phe Cys
   1 5
<210> 58
   <211> 11
   <212> PRT
   <213> Artificial Sequence
<220>
   <223> Description of Artificial Sequence: Peptide
<400> 58
   Ala Cys Asp Cys Arg Gly Asp Cys Phe Cys Gly
   1 5 10
<210> 59
   <211> 6
   <212> PRT
   <213> Artificial Sequence
<220>
   <223> Description of Artificial Sequence: Peptide
<400> 59
   Gly Arg Gly Glu Ser Pro
   1 5

### SEQUENCE LISTING

<110> La Jolla Cancer Research Foundation
<120> Molecules that home to a selected organ or tissue in vivo and methods of identifying same
<130> P052211
<140>
   <141>
<150> US 08/526, 708
   <151> 1995-09-11
<150> US 08/526,710
   <151> 1995-09-11
<160> 59
<170> PatentIn Ver. 2.1
<210> 1
   <211> 9
   <212> PRT
   <213> Artificial Sequence
<220>
   <223> Description of Artificial Sequence: Peptide
<400> 1
   Cys Asn Ser Arg Leu His Leu Arg Cys
     1               5
<210> 2
   <211> 9
   <212> PRT
   <213> Artificial Sequence
<220>
   <223> Description of Artificial Sequence: Peptide
<400> 2
   Cys Glu Asn Trp Trp Gly Asp Val Cys
     1               5
<210> 3
   <211> 9
   <212> PRT
   <213> Artificial Sequence
<220>
   <223> Description of Artificial Sequence: Peptide
<400> 3
   Cys Leu Ser Ser Arg Leu Asp Ala Cys
     1               5
<210> 4
   <211> 8
   <212> PRT
   <213> Artificial Sequence
<220>
   <223> Description of Artificial Sequence: Peptide
<400> 4
   Cys Val Leu Arg Gly Gly Arg Cys
     1               5
<210> 5
   <211> 9
   <212> PRT
   <213> Artificial Sequence
<220>
   <223> Description of Artificial Sequence: Peptide
<400> 5
   Cys Asn Ser Arg Leu Gln Leu Arg Cys
   1 5
<210> 6
   <211> 7
   <212> PRT
   <213> Artificial Sequence
<220>
   <223> Description of Artificial Sequence: Peptide
<400> 6
   Cys Gly Val Arg Leu Gly Cys
   1 5
<210> 7
   <211> 8
   <212> PRT
   <213> Artificial Sequence
<220>
   <223> Description of Artificial Sequence: Peptide
<400> 7
   Cys Lys Asp Trp Gly Arg Ile Cys
   1 5
<210> 8
   <211> 8
   <212> PRT
   <213> Artificial Sequence
<220>
   <223> Description of Artificial Sequence: Peptide
<400> 8
   Cys Leu Asp Trp Gly Arg Ile Cys
   1 5
<210> 9
   <211> 8
   <212> PRT
   <213> Artificial Sequence
<220>
   <223> Description of Artificial Sequence: Peptide
<400> 9
   Cys Thr Arg Ile Thr Glu Ser Cys
    1               5
<210> 10
   <211> 7
   <212> PRT
   <213> Artificial Sequence
<220>
   <223> Description of Artificial Sequence: Peptide
<400> 10
   Cys Glu Thr Leu Pro Ala Cys
    1               5
<210> 11
   <211> 8
   <212> PRT
   <213> Artificial Sequence
<220>
   <223> Description of Artificial Sequence: Peptide
<400> 11
   Cys Arg Thr Gly Thr Leu Phe Cys
    1               5
<210> 12
   <211> 8
   <212> PRT
   <213> Artificial Sequence
<220>
   <223> Description of Artificial Sequence: Peptide
<400> 12
   Cys Gly Arg Ser Leu Asp Ala Cys
    1               5
<210> 13
   <211> 9
   <212> PRT
   <213> Artificial Sequence
<220>
   <223> Description of Artificial Sequence: Peptide
<400> 13
   Cys Arg His Trp Phe Asp Val Val Cys
    1               5
<210> 14
   <211> 8
   <212> PRT
   <213> Artificial Sequence
<220>
   <223> Description of Artificial Sequence: Peptide
<400> 14
   Cys Ala Asn Ala Gln Ser His Cys
    1               5
<210> 15
   <211> 8
   <212> PRT
   <213> Artificial Sequence
<220>
   <223> Description of Artificial Sequence: Peptide
<400> 15
   Cys Gly Asn Pro Ser Tyr Arg Cys
    1               5
<210> 16
   <211> 21
   <212> PRT
   <213> Artificial Sequence
<220>
   <223> Description of Artificial Sequence: Peptide
<400> 16
   Trp Arg Cys Val Leu Arg Glu Gly Pro Ala Gly Gly Cys Ala Trp Phe
    1               5                  10                  15
   Asn Arg His Arg Leu
                20
<210> 17
   <211> 20
   <212> PRT
   <213> Artificial Sequence
<220>
   <223> Description of Artificial Sequence: Peptide
<400> 17
   Tyr Pro Cys Gly Gly Glu Ala Val Ala Gly Val Ser Ser Val Arg Thr
    1               5                  10                  15
   Met Cys Ser Glu
                20
<210> 18
   <211> 20
   <212> PRT
   <213> Artificial Sequence
<220>
   <223> Description of Artificial Sequence: Peptide
<400> 18
   Leu Asn Cys Asp Tyr Gln Gly Thr Asn Pro Ala Thr Ser Val Ser Val
    1               5                  10                  15
   Pro Cys Thr Val
                20
<210> 19
   <211> 7
   <212> PRT
   <213> Artificial Sequence
<220>
   <223> Description of Artificial Sequence: Peptide
<400> 19
   Leu Ser Ser Arg Leu Asp Ala
    1               5
<210> 20
   <211> 9
   <212> PRT
   <213> Artificial Sequence
<220>
   <223> Description of Artificial Sequence: Peptide
<400> 20
   Val Leu Arg Glu Gly Pro Ala Gly Gly
    1               5
<210> 21
   <211> 7
   <212> PRT
   <213> Artificial Sequence
<220>
   <223> Description of Artificial Sequence: Peptide
<400> 21
   Cys Leu Pro Val Ala Ser Cys
   1 5
<210> 22
   <211> 7
   <212> PRT
   <213> Artificial Sequence
<220>
   <223> Description of Artificial Sequence: Peptide
<400> 22
   Cys Gly Ala Arg Glu Met Cys
   1 5
<210> 23
   <211> 8
   <212> PRT
   <213> Artificial Sequence
<220>
   <223> Description of Artificial Sequence: Peptide
<400> 23
   Cys Lys Gly Arg Ser Ser Ala Cys
   1 5
<210> 24
   <211> 8
   <212> PRT
   <213> Artificial Sequence
<220>
   <223> Description of Artificial Sequence: Peptide
<400> 24
   Cys Trp Ala Arg Ala Gln Gly Cys
   1 5
<210> 25
   <211> 8
   <212> PRT
   <213> Artificial Sequence
<220>
   <223> Description of Artificial Sequence: Peptide
<400> 25
   Cys Leu Gly Arg Ser Ser Val Cys
   1 5
<210> 26
   <211> 8
   <212> PRT
   <213> Artificial Sequence
<220>
   <223> Description of Artificial Sequence: Peptide
<400> 26
   Cys Thr Ser Pro Gly Gly Ser Cys
   1 5
<210> 27
   <211> 8
   <212> PRT
   <213> Artificial Sequence
<220>
   <223> Description of Artificial Sequence: Peptide
<400> 27
   Cys Met Gly Arg Trp Arg Leu Cys
   1 5
<210> 28
   <211> 8
   <212> PRT
   <213> Artificial Sequence
<220>
   <223> Description of Artificial Sequence: Peptide
<400> 28
   Cys Val Gly Glu Cys Gly Gly Cys
   1 5
<210> 29
   <211> 7
   <212> PRT
   <213> Artificial Sequence
<220>
   <223> Description of Artificial Sequence: Peptide
<400> 29
   Cys Val Ala Trp Leu Asn Cys
   1 5
<210> 30
   <211> 7
   <212> PRT
   <213> Artificial Sequence
<220>
   <223> Description of Artificial Sequence: Peptide
<400> 30
   Cys Arg Arg Phe Gln Asp Cys
   1 5
<210> 31
   <211> 7
   <212> PRT
   <213> Artificial Sequence
<220>
   <223> Description of Artificial Sequence: Peptide
<400> 31
   Cys Leu Met Gly Val His Cys
   1 5
<210> 32
   <211> 8
   <212> PRT
   <213> Artificial Sequence
<220>
   <223> Description of Artificial Sequence: Peptide
<400> 32
   Cys Lys Leu Leu Ser Gly Val Cys
   1 5
<210> 33
   <211> 8
   <212> PRT
   <213> Artificial Sequence
<220>
   <223> Description of Artificial Sequence: Peptide
<400> 33
   Cys Phe Val Gly His Asp Leu Cys
   1 5
<210> 34
   <211> 7
   <212> PRT
   <213> Artificial Sequence
<220>
   <223> Description of Artificial Sequence: Peptide
<400> 34
   Cys Arg Cys Leu Asn Val Cys
   1 5
<210> 35
   <211> 7
   <212> PRT
   <213> Artificial Sequence
<220>
   <223> Description of Artificial Sequence: Peptide
<400> 35
   Cys Lys Leu Met Gly Glu Cys
   1 5
<210> 36
   <211> 7
   <212> PRT
   <213> Artificial Sequence
<220>
   <223> Description of Artificial Sequence: Peptide
<400> 36
   Cys Xaa Xaa Xaa Xaa Xaa Cys
   1 5
<210> 37
   <211> 8
   <212> PRT
   <213> Artificial Sequence
<220>
   <223> Description of Artificial Sequence: Peptide
<400> 37
   Cys Xaa Xaa Xaa Xaa Xaa Xaa Cys
   1               5
<210> 38
   <211> 9
   <212> PRT
   <213> Artificial Sequence
<220>
   <223> Description of Artificial Sequence: Peptide
<400> 38
   Cys Xaa Xaa Xaa Xaa Xaa Xaa Xaa Cys
   1               5
<210> 39
   <211> 10
   <212> PRT
   <213> Artificial Sequence
<220>
   <223> Description of Artificial Sequence: Peptide
<400> 39
   Cys Xaa Xaa Xaa Xaa Xaa Xaa Xaa Xaa Xaa
   1               5                   10
<210> 40
   <211> 20
   <212> PRT
   <213> Artificial Sequence
<220>
   <223> Description of Artificial Sequence: Peptide
<400> 40
   Xaa Xaa Cys Xaa Xaa Xaa Xaa Xaa Xaa Xaa Xaa Xaa Xaa Xaa Xaa Xaa
   1               5                   10                  15
   Xaa Cys Xaa Xaa
               20
<210> 41
   <211> 21
   <212> PRT
   <213> Artificial Sequence
<220>
   <223> Description of Artificial Sequence: Peptide
<400> 41
   Xaa Xaa Cys Xaa Xaa Xaa Xaa Xaa Xaa Xaa Xaa Xaa Xaa Xaa Xaa Xaa
   1 5 10 15
   Xaa Xaa Xaa Xaa Xaa
   20
<210> 42
   <211> 21
   <212> DNA
   <213> Artificial Sequence
<220>
   <223> Description of Artificial Sequence: Oligonucleotide
<400> 42
   tgtnnknnkn nknnknnktg t 21
<210> 43
   <211> 9
   <212> PRT
   <213> Artificial Sequence
<220>
   <223> Description of Artificial Sequence: Peptide
<400> 43
   Cys Val Arg Leu Asn Ser Leu Ala Cys
   1 5
<210> 44
   <211> 4
   <212> PRT
   <213> Artificial Sequence
<220>
   <223> Description of Artificial Sequence: Peptide
<400> 44
   Asp Xaa Xaa Arg
   1
<210> 45
   <211> 4
   <212> PRT
   <213> Artificial Sequence
<220>
   <223> Description of Artificial Sequence: Peptide
<220>
   <223> Xaa at position 1 is about 1 to about 10 independently selected amino acids
<220>
   <223> Xaa at position 4 is about 1 to about 10 independently selected amino acids
<400> 45
   Xaa Ser Arg Xaa
   1
<210> 46
   <211> 5
   <212> PRT
   <213> Artificial Sequence
<220>
   <223> Description of Artificial Sequence: Peptide
<220>
   <223> Xaa at position 1 is absent or is about 1 to about 10 independently selected amino acids
<220>
   <223> Xaa at position 5 is about 1 to about 20 independently selected amino acids
<400> 46
   Xaa Val Leu Arg Xaa
   1 5
<210> 47
   <211> 12
   <212> PRT
   <213> Artificial Sequence
<220>
   <223> Description of Artificial Sequence: Peptide
<400> 47
   Cys Xaa Xaa Cys Xaa Xaa Xaa Cys Xaa Xaa Xaa Cys
   1 5 10
<210> 48
   <211> 13
   <212> PRT
   <213> Artificial Sequence
<220>
   <223> Description of Artificial Sequence: Peptide
<400> 48
   Cys Gly Arg Glu Cys Pro Arg Leu Cys Gln Ser Ser Cys
   1 5 10
<210> 49
   <211> 13
   <212> PRT
   <213> Artificial Sequence
<220>
   <223> Description of Artificial Sequence: Peptide
<400> 49
   Cys Gly Glu Ala Cys Gly Gly Gln Cys Ala Leu Pro Cys
    1               5                  10
<210> 50
   <211> 13
   <212> PRT
   <213> Artificial Sequence
<220>
   <223> Description of Artificial Sequence: Peptide
<400> 50
   Cys Asn Gly Arg Cys Val Ser Gly Cys Ala Gly Arg Cys
    1               5                  10
<210> 51
   <211> 13
   <212> PRT
   <213> Artificial Sequence
<220>
   <223> Description of Artificial Sequence: Peptide
<400> 51
   Cys Gly Leu Met Cys Gln Gly Ala Cys Phe Asp Val Cys
    1               5                  10
<210> 52
   <211> 6
   <212> PRT
   <213> Artificial Sequence
<220>
   <223> Description of Artificial Sequence: Peptide
<400> 52
   Trp Gly Thr Gly Leu Cys
    1               5
<210> 53
   <211> 7
   <212> PRT
   <213> Artificial Sequence
<220>
   <223> Description of Artificial Sequence: Peptide
<400> 53
   Cys Ser Val Ala Asn Ser Cys
   1 5
<210> 54
   <211> 7
   <212> PRT
   <213> Artificial Sequence
<220>
   <223> Description of Artificial Sequence: Peptide
<400> 54
   Cys Ser Ser Thr Met Arg Cys
   1 5
<210> 55
   <211> 8
   <212> PRT
   <213> Artificial Sequence
<220>
   <223> Description of Artificial Sequence: Peptide
<400> 55
   Cys Leu Ser Gly Ser Leu Ser Cys
   1 5
<210> 56
   <211> 9
   <212> PRT
   <213> Artificial Sequence
<220>
   <223> Description of Artificial Sequence: Peptide
<400> 56
   Cys Ile Glu Gly Val Leu Gly Gly Cys
   1 5
<210> 57
   <211> 9
   <212> PRT
   <213> Artificial Sequence
<220>
   <223> Description of Artificial Sequence: Peptide
<400> 57
   Cys Asp Cys Arg Gly Asp Cys Phe Cys
   1 5
<210> 58
   <211> 11
   <212> PRT
   <213> Artificial Sequence
<220>
   <223> Description of Artificial Sequence: Peptide
<400> 58
   Ala Cys Asp Cys Arg Gly Asp Cys Phe Cys Gly
   1 5 10
<210> 59
   <211> 6
   <212> PRT
   <213> Artificial Sequence
<220>
   <223> Description of Artificial Sequence: Peptide
<400> 59
   Gly Arg Gly Glu Ser Pro
   1 5

## Description

### BACKGROUND OF THE INVENTION

### FIELD OF THE INVENTION

The present invention relates generally to the fields of molecular medicine and drug delivery and, more specifically, to molecules that home to breast tumors, peptides having the amino acid sequence of SEQ ID NO: 48, 49 or 50.

### BACKGROUND INFORMATION

Although the effect of a particular pathology often is manifest throughout the body of the afflicted person, generally, the underlying pathology may affect only a single organ or tissue. In many cases, drugs are the treatment of choice for a patient suffering a particular disease. It is rare, however, that a drug will target only the diseased tissue or organ. More commonly, drug treatment results in undesirable side effects due, for example, to generalized toxic effects throughout the patient's body. The nausea, loss of hair and drop in blood count that occur as a result of treating of a cancer patient with chemotherapeutic agents are examples of the undesirable side effects that can occur due to drug treatment.

The undesirable side effects that can occur when drugs are used to treat a disease most often are due to the inability of the drug to specifically target the diseased organ or tissue. For example, a cancer chemotherapeutic agent that targets rapidly proliferating cells would be useful to kill rapidly dividing cancer cells. However, such an agent also kills normal proliferating hematopoietic and epithelial cells. Thus, the dose of such a drug that can be administered to a patient is limited due to its toxic effect on normal cells.

Efforts have been made to increase the target specificity of various drugs. In some cases, a particular cell type present in a diseased tissue or organ may express a unique cell surface marker. In such a case, an antibody can be raised against the unique cell surface marker and a drug can be linked to the antibody. Upon administration of the drug/antibody complex to the patient, the binding of the antibody to the cell surface marker results in the delivery of a relatively high concentration of the drug to the diseased tissue or organ. Similar methods can be used where a particular cell type in the diseased organ expresses a unique cell surface receptor or a ligand for a particular receptor. In these cases, the drug can be linked to the specific ligand or to the receptor, respectively, thus providing a means to deliver a relatively high concentration of the drug to the diseased organ.

While linking a drug to a molecule that homes to a particular cell type present in a diseased organ or tissue provides significant advantages for treatment over the use of a drug, alone, use of this method is severely limited. In particular, very few cell type specific antibodies have been described and it can be difficult and time consuming to attempt to obtain an antibody that targets an organ in a particular patient suffering a pathology. Furthermore, few cell type specific surface markers have been described. Even where such markers have been described, the cells expressing the markers can be distributed among various tissues or organs, thereby limiting their usefulness as targets. Thus, it is important to identify specific target cell markers that are expressed in only one or a few tissues or organs and to identify molecules that specifically interact with such markers.

Various cell types can express unique markers and, therefore, provide potential targets for organ homing molecules. Endothelial cells, for example, which line the internal surfaces of blood vessels, can have distinct morphologies and biochemical markers in different tissues. The blood vessels of the lymphatic system, for example, express various adhesion proteins that serve to guide lymphocyte homing. In addition, endothelial cells present in lymph nodes express a cell surface marker that is a ligand for L-selectin and endothelial cells in Peyer's patch venules express a ligand for the α₄β₇ integrin. These ligands are involved in specific lymphocyte homing to their respective lymphoid organs. Thus, linking a drug to L-selectin or to the α₄β₇ integrin may provide a means for targeting the drug to diseased lymph nodes or Peyer's patches, respectively, provided that these molecules do not bind to similar ligands present in a significant number of other organs.

WO 95/14714 discloses peptides that selectively bind to integrins.

Although the homing molecules present in the blood vessels of non-lymphoid tissues have not been clearly defined, the ability of lymphocytes to return to the organ in which they were first stimulated indicates that organ-specific endothelial markers exist. Similarly, the homing or metastasis of particular types of tumor cells to specific organs provides further evidence that organ-specific markers exist. However, there remains a need to identify other organ-specific cell markers and the molecules that bind to them.

Methods are now available for producing large populations of molecules and for screening libraries of molecules to identify those of interest. For example, phage peptide display libraries can be used to express large numbers of peptides that can be screened *in vitro* with a particular target molecule or a cell of interest in order to identify peptides that specifically bind the target molecule or the cell. Screening of such phage display libraries has been used, for example, to identify ligands that specifically bind various antibodies and cell surface receptors.

Screening of a phage display library generally involves *in vitro* panning of the library using a purified target molecule. Phage that bind the target molecule can be recovered, individual phage can be cloned and the peptide expressed by a cloned phage can be determined. Such a peptide can be useful for delivery of a drug linked to the peptide to cells expressing the target molecule.

Unfortunately, very few target molecules that are expressed by only one or a few cell types have been identified. Furthermore, even where such a target molecule is known, it is uncertain whether a peptide that specifically binds the target molecule, as determined using an *in vitro* panning method, will bind to the target molecule *in vivo.* As a result, the identification of a peptide from a phage display library using an *in vitro* panning method essentially represents only a starting point for determining whether the identified peptide can be useful for an *in vivo* procedure. Thus, a need exists to develop *in vivo* methods for screening large numbers of molecules such as peptides in order to identify those that can home to one or more selected organs or tissues. The present invention satisfies this need and provides related advantages as well.

### SUMMARY OF THE INVENTION

The present invention provides peptides that selectively home to a selected organ or tissue. The invention provides the tumor homing peptides CGRECPRLCQSSC (SEQ ID NO: 48), CGEACGGQCALPC (SEQ ID NO: 49) and CNGRCVSGCAGRC (SEQ ID NO: 50) which home to a breast tumor. Such organ or tissue homing molecules are referred to herein, for convenience, as "organ homing" molecules. A "tumor homing molecule" is an example of an organ homing molecule. An organ homing molecule of the invention is useful, for example, for targeting a moiety such as a drug to a breast tumor in vitro, for preparing a pharmaceutical composition for directing a moiety to a breast tumor, or for identifying the presence of a target molecule in a sample.

Information regarding further organ homing molecules and methods used to identify the molecules is included as technical background.

### DETAILED DESCRIPTION OF THE INVENTION

For identifying the molecules of the invention, the present invention employed a method of identifying a molecule that homes specifically to a breast cancer, by screening a library using *in vivo* panning (see Pasqualini and Ruoslahti, Nature 380:364-366 (1996). The identified organ homing molecules are useful, for example, for targeting a desired moiety such as a drug, a toxin or a detectable label, which can be linked to the molecule, to the selected organ or tissue. *In vivo* panning provides a direct means for identifying molecules that selectively home to a selected organ or tissue and, therefore, provides a significant advantage over previous methods, which require that a molecule identified using an *in vitro* screening method subsequently be examined to determine whether it maintains its specificity *in vivo.*

As used herein, the term "library" means a collection of molecules. A library can contain a few or a large number of different molecules, varying from about ten molecules to several billion molecules or more. If desired, a molecule can be linked to a tag, which can be a shared tag or a specific tag that can facilitate recovery or identification of the molecule.

As used herein, the term "molecule" is used broadly to mean an organic chemical such as a drug; a peptide, including a variant or modified peptide or peptide-like molecules such as a peptidomimetic or peptoid; or a protein such as an antibody or a growth factor receptor or a fragment thereof such as an Fv, Fd or Fab fragment of an antibody, which contains a binding domain. For convenience, the term "peptide" is used broadly herein to mean peptides, proteins, fragments of proteins and the like. A molecule can be a non-naturally occurring molecule, which does not occur in nature, but is produced as a result of *in vitro* methods, or can be a naturally occurring molecule such as a protein or fragment thereof expressed from a cDNA library.

Methods for preparing libraries containing diverse populations of various types of molecules such as peptides, peptoids and peptidomimetics are well known in the art and commercially available (see, for example, Ecker and Crooke, Biotechnology 13:351-360 (1995), and Blondelle et al., Trends Anal. Chem. 14:83-92 (1995), and the references cited therein, each of which is incorporated herein by reference; see, also, Goodman and Ro, Peptidomimetics for Drug Design, in "Burger's Medicinal Chemistry and Drug Discovery" Vol. 1 (ed. M.E. Wolff; John Wiley & Sons 1995), pages 803-861, and Gordon et al., J. Med. Chem. 37:1385-1401 (1994). Where a molecule is a peptide, protein or fragment thereof, the molecule can be produced *in vitro* directly or can be expressed from a nucleic acid, which is produced *in vitro.* Methods of synthetic peptide and nucleic acid chemistry are well known in the art.

A library of molecules also can be produced, for example, by constructing a cDNA expression library from mRNA collected from a cell, tissue, organ or organism of interest. Methods for producing such libraries are well known in the art (see, for example, Sambrook et al., Molecular Cloning: A laboratory manual (Cold Spring Harbor Laboratory Press 1989). Preferably, the peptide encoded by the cDNA is expressed on the surface of a cell or a virus containing the cDNA. For example, cDNA can be cloned into a phage vector such as fuse 5 (see Example I), wherein, upon expression, the encoded peptide is expressed as a fusion protein on the surface of the phage.

In addition, a library of molecules can comprise a library of nucleic acid molecules. Nucleic acid molecules that bind, for example, to a cell surface receptor are known in the art (see O'Connell et al., Proc. Natl. Acad. Sci., USA 93:5883-5887 (1996); Tuerk and Gold, Science 249:505-510 (1990); Gold et al., Ann. Rev. Biochem. 64:763-797 (1995). Thus, a library of nucleic acid molecules can be administered to a subject having a tumor and tumor homing molecules can be identified by *in vivo* panning as disclosed herein. If desired, the nucleic acid molecules can be nucleic acid analogs that, for example, are less susceptible to attack by nucleases (see, for example, Jelinek et al., Biochemistry 34:11363-11372 (1995); Latham et al., Nucl. Acids Res. 22:2817-2822 (1994); Tam et al., Nucl. Acids Res. 22:977-986 (1994); Reed et al., Cancer Res. 59:6565-6570 (1990).

As disclosed herein, *in vivo* panning comprises administering a library to a subject, collecting a selected organ or tissue and identifying an organ homing molecule. An organ homing molecule can be identified using various methods well known in the art. Generally, the presence of an organ homing molecule in a selected organ or tissue is identified based on one or more characteristics common to the molecules present in the library, then the structure of a particular organ homing molecule is identified. For example, a highly sensitive detection method such as mass spectrometry, either alone or in combination with a method such as gas chromatography, can be used to identify organ homing molecules in a selected organ or tissue. Thus, where a library comprises diverse molecules based generally on the structure of an organic molecule, the presence of an organ homing molecule such as a drug can be identified by detecting the presence of a parent peak for the particular molecule.

If desired, the selected organ or tissue can be collected, then processed using a method such as HPLC, which can provide a fraction enriched in molecules having a defined range of molecular weights or polar or nonpolar characteristics or the like. Conditions for HPLC will depend on the chemistry of the particular molecule and are well known to those skilled in the art. Similarly, methods for bulk removal of potentially interfering cellular materials such as DNA, RNA, proteins, lipids or carbohydrates are well known in the art as are methods for enriching a fraction containing an organic molecule using, for example, methods of selective extraction. For example, where a library comprises a population of diverse organic chemical molecules each linked to a specific oligonucleotide tag, such that the particular molecule is identified by determining the oligonucleotide sequence using the polymerase chain reaction (PCR), genomic DNA can be removed from the sample of the collected organ in order to reduce the potential for background PCR reactions. In addition, a library can comprise a population of diverse molecules such as organic chemical molecules each linked to a common, shared tag. Based on the presence and properties of the shared tag, molecules of the library that selectively home to an organ or tissue can be substantially isolated from a sample of the organ or tissue. These and other methods can be useful for enriching the sample of the collected organ for the particular organ homing molecule, thereby removing potentially contaminating materials from the collected organ sample and increasing the sensitivity of detecting the molecule.

Evidence provided herein indicates that a sufficient number of organ homing molecules selectively home to a selected organ or tissue during *in vivo* panning such that the molecules readily can be identified. For example, various independent phage expressing the same peptide were identified in a tumor formed from implanted breast carcinoma cells (Table 3). Specifically, one peptide that homed to the breast tumor constituted about 43% of the sequenced breast tumor homing peptides.

Although a substantial fraction of the identified organ homing molecules have the same structure, the peptide inserts of only a small number of isolated phage were determined. It should be recognized, however, that hundreds of thousands to millions of phage expressing organ homing peptides were recovered following various *in vivo* pannings for organ homing molecules. These results indicate that specific organ homing molecules will be present in substantial numbers in an organ following *in vivo* homing, thereby increasing the ease with which the molecules can be identified.

Ease of identification of an organ homing molecule, particularly an untagged molecule, depends on various factors, including the presence of potentially contaminating background cellular material. Thus, where the organ homing molecule is an untagged peptide, a larger number must home to the organ in order to identify the specific peptides against the background of cellular protein. In contrast, a much smaller number of an untagged organic chemical homing molecule is identifiable because such molecules normally are absent from or present in only small numbers in the body. In such a case, a highly sensitive method such as mass spectrometry can be used to identify an organ homing molecule. The skilled artisan will recognize that the method of identifying a molecule will depend, in part, on the chemistry of the particular molecule.

Where an organ homing molecule is a nucleic acid or is tagged with a nucleic acid molecule, an assay such as PCR can be particularly useful for identifying the presence of the molecule because, in principle, PCR can detect the presence of a single nucleic acid molecule (see, for example, Erlich, PCR Technology: Principles and Applications for DNA Amplification (Stockton Press 1989). Preliminary studies have demonstrated that, following intravenous injection of 10 ng of an approximately 6000 base pair plasmid into a mouse and 2 minutes in the circulation, the plasmid was detectable by PCR in a sample of lung. These results indicate that nucleic acids are sufficiently stable when administered into the circulation such that *in vivo* panning can be used to identify nucleic acid molecules that selectively home to a selected organ.

The molecules of a library can be tagged, which can facilitate recovery or identification of the molecule. As used herein, the term "tag" means a physical, chemical or biological moiety such as a plastic microbead, an oligonucleotide or a bacteriophage, respectively, that is linked to a molecule of the library. Methods for tagging a molecule are well known in the art (Hermanson, Bioconjugate Techniques (Academic Press 1996).

A tag, which can be a shared tag or a specific tag, can be useful for identifying the presence or structure of a molecule of a library. As used herein, the term "shared tag" means a physical, chemical or biological moiety that is common to each molecule in a library. Biotin, for example, can be a shared tag that is linked to each molecule in a library. A shared tag can be useful to identify the presence of a molecule of the library in a sample and also can be useful to substantially isolate the molecules from a sample. For example, where the shared tag is biotin, the biotin-tagged molecules in a library can be substantially isolated by binding to streptavidin or their presence can be identified by binding with a labeled streptavidin. Where a library is a phage display library, the phage that express the peptides are another example of a shared tag, since each peptide of the library is linked to a phage. In addition, a peptide such as the hemaglutinin antigen can be a shared tag that is linked to each molecule in a library, thereby allowing the use of an antibody specific for the hemaglutinin antigen to substantially isolate molecules of the library from a sample of a selected organ or tissue.

A shared tag also can be a nucleic acid sequence that can be useful to identify the presence of molecules of the library in a sample or to substantially isolate molecules of a library from a sample. For example, each of the molecules of a library can be linked to the same selected nucleotide sequence, which constitutes the shared tag. An affinity column containing a nucleotide sequence that is complementary to the shared tag then can be used to hybridize molecules of the library containing the shared tag, thus substantially isolating the molecules from an organ or tissue sample. A nucleotide sequence complementary to a portion of the shared nucleotide sequence tag also can be used as a PCR primer such that the presence of molecules containing the shared tag can be identified in a sample by PCR.

A tag also can be a specific tag. As used herein, the term "specific tag" means a physical, chemical or biological tag that is linked to a particular molecule in a library and is unique for that particular molecule. A specific tag is particularly useful if it is readily identifiable. A nucleotide sequence that is unique for a particular molecule of a library is an example of a specific tag. For example, the method of synthesizing peptides tagged with a unique nucleotide sequence provides a library of molecules, each containing a specific tag, such that upon determining the nucleotide sequence, the identity of the peptide is known (see Brenner and Lerner, Proc. Natl. Acad. Sci., USA 89:5381-5383 (1992). The use of a nucleotide sequence as a specific tag for a peptide or other type of molecule provides a simple means to identify the presence of the molecule in a sample because an extremely sensitive method such as PCR can be used to determine the nucleotide sequence of the specific tag, thereby identifying the sequence of the molecule linked thereto. Similarly, the nucleic acid sequence encoding a peptide expressed on a phage is another example of a specific tag, since sequencing of the specific tag identifies the amino acid sequence of the expressed peptide.

The presence of a shared tag or a specific tag can provide a means to identify or recover an organ homing molecule of the invention following *in vivo* panning. In addition, the combination of a shared tag and specific tag can be particularly useful for identifying an organ homing molecule. For example, a library of peptides can be prepared such that each is linked to a specific nucleotide sequence tag (see, for example, Brenner and Lerner, *supra,* 1992), wherein each specific nucleotide sequence tag has incorporated therein a shared tag such as biotin. Upon homing to an organ, the particular organ homing peptides can be substantially isolated from a sample of the organ based on the shared tag and the specific peptides can be identified, for example, by PCR of the specific tag (see Erlich, *supra,* 1989).

A tag also can serve as a support. As used herein, the term "support" means a tag having a defined surface to which a molecule can be attached. In general, a tag useful as a support is a shared tag. For example, a support can be a biological tag such as a virus or virus-like particle such as a bacteriophage ("phage"); a bacterium such as *E. coli*; or a eukaryotic cell such as a yeast, insect or mammalian cell; or can be a physical tag such as a liposome or a microbead, which can be composed of a plastic, agarose, gelatin or other material. If desired, a shared tag useful as a support can have linked thereto a specific tag. Thus, the phage display libraries used in the exemplified methods can be considered to consist of the phage, which is a shared tag that also is a support, and the nucleic acid sequence encoding the expressed peptide, the nucleic acid sequence being a specific tag.

In general, a support should have a diameter less than about 10 µm to about 50 µm in its shortest dimension, such that the support can pass relatively unhindered through the capillary beds present in the subject and not occlude circulation. In addition, a support can be nontoxic, so that it does not perturb the normal expression of cell surface molecules or normal physiology of the subject, and biodegradable, particularly where the subject used for *in vivo* panning is not sacrificed to collect a selected organ or tissue.

Where a molecule is linked to a support, the tagged molecule comprises the molecule attached to the surface of the support, such that the part of the molecule suspected of being able to interact with a target in a cell in the subject is positioned so as to be able to participate in the interaction. For example, where the molecule is suspected of being a β adrenergic agonist, the binding portion of the molecule attached to a support is positioned so it can interact with a β adrenergic receptor on a cell in the selected organ or tissue. Similarly, where the molecule is suspected of being a ligand for a growth factor receptor, the molecule is positioned on the support so that it can bind the receptor. If desired, an appropriate spacer molecule can be positioned between the molecule and the support such that the ability of the potential organ homing molecule to interact with the target molecule is not hindered. A spacer molecule also can contain a reactive group, which provides a convenient and efficient means of linking a molecule to a support and, if desired, can contain a tag, which can facilitate recovery or identification of the molecule (see Hermanson, *supra,* 1996).

As exemplified herein, a peptide suspected of being able to home to a selected organ or tissue such as brain was expressed as the N-terminus of a fusion protein, wherein the C-terminus consisted of a phage coat protein. Upon expression of the fusion protein, the C-terminal coat protein linked the fusion protein to the surface of a phage such that the N-terminal peptide was in a position to interact with a target molecule in an organ. Thus, a molecule having a shared tag was formed by the linking of a peptide to a phage, wherein the phage provided a biological support, the peptide molecule is linked as a fusion protein, the phage-encoded portion of the fusion protein acts as a spacer molecule, and the nucleic acid encoding the peptide provided a specific tag allowing identification of an organ homing peptide.

As used herein, the term "*in vivo* panning" means a method of screening a library by administering the library to a subject and identifying a molecule that selectively homes to one or a few selected organs or tissues. The term "administering to a subject", when used in referring to a library of molecules or a portion thereof, is used in its broadest sense to mean that the library is delivered to a selected organ or tissue, including, where desired, a tumor, such that the molecules of the library contact the selected organ or tissue.

A library can be administered to a subject, for example, by injecting the library into the circulation of the subject such that the molecules can pass through the selected organ or tissue; after an appropriate period of time, circulation is terminated by sacrificing the subject or by removing a sample of the organ (see Example I). Alternatively, a cannula can be inserted into a blood vessel in the subject, such that the library is administered by perfusion for an appropriate period of time, after which the library can be removed from the circulation through the cannula or the subject can be sacrificed or the organ can be sampled to terminate circulation. Similarly, a library can be shunted through one or a few organs by cannulation of the appropriate blood vessels in the subject. It is recognized that a library also can be administered to an isolated perfused organ. Such panning in an isolated perfused organ can be useful for identifying molecules that bind to the organ and, if desired, can be used as an initial screening of a library. For example, if a kidney homing molecule is desired, a library can be perfused through an isolated kidney, then the molecules that bound to the perfused kidney can be screened by *in vivo* panning to identify a kidney homing molecule.

The *in vivo* panning method is exemplified herein by screening a phage peptide display library in mice and identifying specific peptides that selectively home to brain, kidney or a tumor (see Examples II and III).

Phage display technology provides a means for expressing a diverse population of random or selectively randomized peptides. Various methods of phage display and methods for producing diverse populations of peptides are well known in the art. For example, Ladner et al. (U.S. Patent No. 5,223,409, issued June 29, 1993) describe methods for preparing diverse populations of binding domains on the surface of a phage. In particular, Ladner et al. describe phage vectors useful for producing a phage display library, as well as methods for selecting potential binding domains and producing randomly or selectively mutated binding domains.

Similarly, Smith and Scott (Meth. Enzymol. 217:228-257 (1993); see, also, Scott and Smith, Science 249: 386-390 (1990) describe methods of producing phage peptide display libraries, including vectors and methods of diversifying the population of peptides that are expressed (see, also, Huse, WO 91/07141 and WO 91/07149, see, also, Example I). Phage display technology can be particularly powerful when used, for example, with a codon based mutagenesis method, which can be used to produce random peptides or randomly or desirably biased peptides (Huse, U.S. Patent No. 5,264,563, *supra,* 1993). These or other well known methods can be used to produce a phage display library, which can be subjected to the *in vivo* panning method employed in the invention in order to identify a peptide that homes to one or a few selected organs or tissues.

In addition, to screening a phage display library, *in vivo* panning can be used to screen various other types of libraries, including, for example, an RNA or cDNA library or a chemical library. If desired, the organ homing molecule can be tagged, which can facilitate recovery of the molecule from a selected organ or tissue or identification of the molecule in the organ or tissue. For example, where a library of organic molecules, each containing a shared tag, is screened, the tag can be a moiety such as biotin, which can be linked directly to the molecule or can be linked to a support containing the molecules. Biotin provides a shared tag useful for recovering the molecule from a selected organ or tissue using an avidin or streptavidin affinity matrix. In addition, a molecule or a support containing a molecule can be linked to a shared tag such as the hapten, 4-ethoxy-methylene-2-phenyl-2-oxazoline-5-one (phOx), which can be bound by an anti-phOx antibody linked to a magnetic bead as a means to recover the tagged molecule. Methods for purifying biotin or phOx tagged molecules are known in the art and the materials for performing these procedures are commercially available (e.g., Invitrogen; La Jolla CA; and Promega Corp.; Madison WI). In the case where a phage library is screened, the phage can be recovered using methods as disclosed in Example I.

*In vivo* panning provides a method for directly identifying molecules that can home to one or a few selected organs or tissues. As used herein, the term "home" or "selectively home" means that a particular molecule binds relatively specifically to a target molecule present in one or few selected organs or tissues following administration to a subject. In general, selective homing is characterized, in part, by detecting at least a two-fold (2x) greater specific binding of the molecule to the selected organ or tissue as compared to a control organ or tissue.

It should be recognized that, in some cases, a molecule can localize nonspecifically to an organ or tissue. For example, *in vivo* panning of a phage display library resulted in a large number of phage localized in organs such as liver, lung and spleen, which contain components of the reticuloendothelial system (RES). Selective homing in such tissues can be distinguished from nonspecific binding by detecting differences, for example, in the abilities of different individual phage to home to a selected organ or tissue, including an organ containing an RES component. For example, selective, homing can be identified by combining a putative organ homing molecule such as a peptide expressed on a phage with a large excess of non-infective phage or with about a five-fold excess of phage expressing unselected peptides, injecting the mixture into a subject and collecting a sample of the selected organ or tissue. In the latter case, for example, provided the number of injected phage expressing organ homing peptides is sufficiently low so as to be nonsaturating for the target molecule, a determination that greater than 20% of the phage in the selected organ or tissue express the putative organ homing molecule is demonstrative evidence that the peptide expressed by the phage is a specific organ homing molecule. In addition, nonspecific localization can be distinguished from selective homing by performing competition experiments as described in Examples II.D. and IV.

Various methods can be useful for preventing nonspecific binding of a molecule to an organ containing a component of the RES. For example, a molecule that homes selectively to such an organ can be obtained by first blocking the RES using a material such as polystyrene latex particles or dextran sulfate (see Kalin et al., Nucl. Med. Biol. 20:171-174 (1993); Illum et al., J. Pharm. Sci. 75:16-22 (1986); Takeya et al., J. Gen. Microbiol. 100: 373-379 (1977), then administering the library to the subject. Thus, Illum et al. administered dextran sulfate 500 or polystyrene microspheres prior to administration of a test substance to block nonspecific uptake of the test substance by Kupffer cells, which are the RES component of the liver (Illum et al., *supra,* 1986). In addition, Takeya et al. reported that the RES in liver could be blocked using carbon particles, dextran sulfate 500 (DS-500) or silica (Takeya et al., *supra,* 1977). Also, Kalin et al. reported that blockage of the RES can be effected using a gelatine colloid (Kalin et al., *supra,* 1993). These and various other agents useful for blocking nonspecific uptake by the RES are known and routinely used.

Undesired binding of phage to RES or to other sites also can be prevented by coinjecting the mice with a specific phage display library together with the same phage made replication-deficient (see Smith et al., *supra,* 1990, 1993). In addition, a peptide that homes to an organ containing an RES component can be identified by preparing a phage display library using phage that exhibit low background binding to such organs. For example, Merrill et al. (Proc. Natl. Acad. Sci., USA 93:3188-3192 (1996) selected lambda-type phage that are not taken up by the RES and, as a result, remain in the circulation for a prolonged period of time. A filamentous phage variant can be selected using similar methods.

As disclosed herein, uptake of peptide-displaying phage by the RES in liver, for example, was blocked by coadministration of non-infective phage, which are not amplifiable. Phage expressing a CX₉ library were injected into mice, either alone or with an excess of non-infective phage (see Example II.E.). Coadministration of non-infective phage with library phage substantially reduced the amount of phage recovered in liver and, to a lesser extent spleen, whereas little or no difference was observed in the number of phage recovered from brain, kidney or lung. These results indicate that uptake of phage by organs such as liver and spleen, which, at least in part can be nonspecific due to the RES component of these organs, can be blocked by coadministration of non-infective phage with a peptide-displaying phage library.

Further experiments were performed to determine whether the phage that were present in liver upon coadministration with non-infective phage had selectively homed to the liver. A phage library was coadministered with non-infective phage, then phage recovered from the liver after a first round of *in vivo* panning were amplified *in vitro* and used for a second round of *in vivo* panning (see Example II.E.). Phage were recovered from liver and from brain (control organ) and quantitated after each round of *in vivo* panning. More phage that homed to liver as compared to brain were recovered following the second round of *in vivo* panning and the population of phage recovered from the liver following the first round of *in vivo* panning was enriched with phage that selectively home to liver. These results demonstrate that the RES component of an organ such as liver can be blocked, thus allowing use of the *in vivo* panning method to identify a molecule that selectively homes to such an organ.

As was observed for liver, a high amount of phage localization also was observed in lung. However, uptake of phage by lung is not nonspecific due, for example, to uptake by alveolar phagocytes, which constitute an RES component in lung. Specifically, phage recovery essentially was the same regardless of whether a phage library was injected alone or was coadministered with non-infective phage (see Example II.E.). Thus, the high uptake of phage observed in the lung (Pasqualini and Ruoslahti, *supra,* 1996) likely is due to the large amount of vasculature in the lungs.

Selective homing can be demonstrated by determining the specificity of an organ homing molecule for the selected organ or tissue as compared to a control organ or tissue. For example, a ratio of brain:kidney homing of up to 9:1 was observed for brain homing peptides (i.e., 9x greater binding to the selected organ as compared to the control organ; see Example II.A.).

Selective homing also can be demonstrated by showing that molecules that home to a selected organ, as identified by one round of *in vivo* panning, are enriched for organ homing molecules in a subsequent round of *in vivo* panning. For example, phage expressing peptides that selectively home to brain were isolated by *in vivo* panning, then were subjected to additional rounds of *in vivo* panning. As demonstrated in Example II.A., phage recovered from brain after a first round of screening showed an 8x enrichment in homing to brain as compared to kidney following a second round of screening and a 13x enrichment following a third round of *in vivo* panning. When a peptide that selectively homes to brain was linked to a moiety, i.e., a red blood cell (RBC), the peptide/RBC complex selectively homed to brain (see Example II.D.).

Due to the conserved nature of cellular receptors and of ligands that bind a particular receptor, the skilled artisan would recognize that an organ homing molecule identified using, for example, *in vivo* panning in a mouse also would bind to the corresponding target molecule in the selected organ or tissue of a human or other species. For example, an RGD-containing peptide that can specifically bind to an integrin expressed by a cell in a human subject also can bind integrins expressed in a variety of species, including integrins expressed in mammalian cells such as murine and bovine cells as well as in cells of more evolutionarily distant species such as *Drosophila.* The ability of an organ homing molecule identified using *in vivo* panning in an experimental animal such as a mouse readily can be examined for the ability to bind to the corresponding organ or tissue in a human subject by demonstrating, for example, that the molecule also can bind specifically *in vitro* to a sample of the selected organ or tissue obtained from a human subject. Thus, routine methods can be used to confirm that an organ homing molecule identified using *in vivo* panning in an experimental animal also can bind an organ-specific target molecule in a human subject. Furthermore, such *in vitro* contacting of an organ homing molecule with a sample suspected of containing a selected organ or tissue can identify the presence of the selected organ or tissue in the sample.

Furthermore, with regard to tumor homing molecules, the skilled artisan would recognize that a tumor homing molecule identified using, for example, *in vivo* panning in a mouse having a murine tumor of a defined histological type such as a melanoma also would bind to the corresponding target molecule in a melanoma in a human or other mammalian species. In addition, a tumor homing molecule that binds a target molecule present in the vasculature in a tumor grown in a mouse likely also can bind to the corresponding target molecule in the vasculature of a tumor in a human or other mammalian subject. A tumor homing,molecule identified using *in vivo* panning in an experimental animal readily can be examined for the ability to bind to a corresponding tumor in a human patient by demonstrating, for example, that the molecule also can bind specifically *in vitro* to a sample of the tumor obtained from the patient. Thus, routine methods can be used to confirm that a tumor homing molecule identified using *in vivo* panning in an experimental animal also can bind a target molecule in a human tumor.

The steps of administering the library to the subject, collecting a selected organ or tissue and identifying the molecules that home to the selected organ or tissue, comprise a single round of *in vivo* panning. Although not required, one or more additional rounds of *in vivo* panning generally are performed. Where an additional round of *in vivo* panning is performed, the molecules recovered from the selected organ or tissue in the previous round are administered to a subject, which can be the same subject used in the previous round, where only a part of the selected organ or tissue was collected.

By performing a second round of *in vivo* panning, the relative binding selectivity of the molecules recovered from the first round can be determined by administering the identified molecules to a subject, collecting the selected organ or tissue, and determining whether more phage is recovered from the organ or tissue as compared to the first round of panning. In addition, if desired, a control organ or tissue can be collected and used as a basis to compare the molecules recovered from the selected organ with those recovered from a control organ. An additional round of *in vivo* panning also can indicate whether the molecules identified from the initially selected organ or tissue also can home to additional organs or tissues, thus defining a family of selected organs or tissues. Additional rounds of panning can be performed as desired.

Ideally, no molecules are recovered from a control organ or tissue following a second or subsequent round of *in vivo* panning. Generally, however, a proportion of the molecules also will be present in a control organ or tissue. In this case, the ratio of molecules in the selected organ as compared to the control organ (selected:control) can be determined. As described above, for example, phage that homed to brain following a first round of *in vivo* panning demonstrated a 13x enrichment in homing to the selected organ as compared to the control organ, kidney, following two additional rounds of panning (Example II).

Additional rounds of *in vivo* panning can be used to determine whether a particular molecule homes only to the selected organ or tissue or can recognize a target on the selected organ that also is expressed in one or more other organs or tissues or is sufficiently similar to the target in the originally selected organ or tissue. Where a molecule is found to direct homing to organs or tissues in addition to the originally selected organ, the organs or tissues are considered to constitute a family of selected organs or tissues. Using the method of *in vivo* panning, molecules that home to only a single selected organ or tissue and molecules that home to a family of selected organs or tissues can be defined. Such identification is expedited by collecting various organs or tissues during subsequent rounds of *in vivo* panning.

As used herein, the term "selected organ or tissue" is used in its broadest sense to mean an organ or a tissue to which a molecule selectively homes. In addition, the term "organ or tissue" is used broadly to mean organ, tissue or cell type, including a cancer cell, in which case the selected organ or tissue can be a tumor such as a primary tumor or a metastatic lesion. In general, a selected organ or tissue contains a cell that expresses a particular target molecule such as a cell surface receptor to which an organ homing molecule can bind. By performing at least two rounds of *in vivo* panning, the selectivity of homing of the molecule to the selected organ or tissue can be determined (see Example II). As discussed above, however, in some cases an organ homing molecule can selectively home to more than one selected organ or tissue, in which case the molecule is considered to be able to selectively home to a family of selected organs or tissues.

The term "control organ or tissue" is used to mean an organ or tissue other than a selected organ. A control organ or tissue is characterized by the inability of an organ homing molecule to home to the control organ or tissue. A control organ or tissue is useful for identifying nonspecific binding of a molecule. A control organ or tissue can be collected, for example, to identify nonspecific binding of the molecule or to determine the selectivity of homing of the molecule (see Examples I and II). In addition, nonspecific binding can be identified by administering, for example, a control molecule, which is known not to home to an organ or tissue but is chemically similar to a potential organ homing molecule. Alternatively, where the administered molecules are linked to a support, administration of the supports, alone, can be used to identify nonspecific binding. For example, a phage that expresses the gene III protein, alone, but that does not contain a peptide fusion protein, can be screened by *in vivo* panning to determine the level of nonspecific binding of the phage support.

In some cases it can take several rounds of *in vivo* panning to identify a control organ or tissue, since a molecule that selectively homes to an originally selected organ also may have the ability to selectively home to additional organs or tissues, thus defining a family of selected organs or tissues. The ability of a molecule to home to one or to a family of selected organs or tissues allows for the preparation of panels of organ homing molecules, wherein individual molecules variously home to one selected organ or tissue or to any of a family of selected organs or tissues with variable selectivity.

In addition, it can be useful to determine whether a molecule that selectively homes to a particular type of tumor also selectively homes to the normal tissue from which the tumor is derived. It can be desirable, for example, to determine whether a molecule that homes to a breast tumor also homes to normal breast tissue. Thus, where *in vivo* panning is used to identify a tumor homing molecule, a sample of the normal tissue counterpart of the tumor can be examined to determine whether homing of the molecule is selective for a target molecule expressed only by the tumor cells or the vascular tissue present in the tumor or is selective for a target molecule normally expressed in the organ or tissue from which the tumor is derived.

In general, a library of molecules, which contains a diverse population of random or selectively randomized molecules of interest, is prepared, then administered to a subject. At a selected time after administration, the subject is sacrificed and a selected organ or tissue or part of the organ or tissue is collected such that the molecules present in the selected organ or tissue can be identified. For example, a mouse was injected with a phage peptide display library, then, after about 1 to 4 minutes, the mouse was anesthetized, frozen in liquid nitrogen to terminate circulation of the phage, a selected organ (brain or kidney; see Examples I and II) or tissue (breast tumor or melanoma; see Example III) was collected, phage present in the selected organ were recovered and peptides that selectively homed to the selected organ or tissue were identified.

In the examples provided, the animals were sacrificed to collect the selected organ or tissue. It should be recognized, however, that only a part of the selected organ need be collected to recover a molecule that homes to that organ. For example, a part of the selected organ or tissue can be collected by biopsy, such that a molecule such as a peptide expressed by a phage, can be administered to the same subject a second time or more, as desired. Where the molecule that is to be administered a second time to the same subject is linked, for example, to a support or to a tag, the support or tag must be nontoxic and should be biodegradable, so as not to interfere with subsequent rounds of screening.

*In vitro* screening of phage libraries previously has been used to identify peptides that bind to antibodies or to cell surface receptors (Smith and Scott, *supra,* 1993). For example, *in vitro* screening of phage peptide display libraries has been used to identify novel peptides that specifically bound to integrin adhesion receptors (Koivunen et al., J. Cell Biol. 124:373-380 (1994a)) and to the human urokinase receptor (Goodson et al., Proc. Natl. Acad. Sci., USA 91:7129-7133 (1994)). However, such *in vitro* studies provide no insight as to whether a peptide that can specifically bind to a selected receptor *in vitro* also will bind the receptor *in vivo* or whether the binding peptide or the receptor are unique to a specific organ in the body. Furthermore, the *in vitro* methods are performed using defined, well-characterized target molecules in an artificial system. For example, Goodson et al. utilized cells expressing a recombinant urokinase receptor. Thus, the *in vitro* methods require prior knowledge of the target molecule and yield little if any information regarding *in vivo* utility. In contrast, the *in vivo* panning method disclosed herein requires no prior knowledge or availability of a target molecule and, therefore, provides a significant advantage over previous methods by identifying molecules that selectively home *in vivo* and the target molecule present in an organ or tissue.

Although a mechanism by which the disclosed method of *in vivo* panning works has not been fully defined, one possibility is that a molecule such as a peptide expressed on a phage recognizes and binds to a target molecule present on endothelial cells lining the blood vessels in a selected organ. Evidence indicates, for example, that the vascular tissues in various organs differ from one another and that such differences can be involved in regulating cellular trafficking in the body. For example, lymphocytes home to lymph nodes or other lymphoid tissues due, in part, to the expression of specific "address" molecules by the endothelial cell in those tissues (Salmi et al., Proc. Natl. Acad. Sci., USA 89:11436-11440 (1992)). Similarly, various leukocytes can recognize sites of inflammation due, in part, to the expression of endothelial cell markers induced by inflammatory signals (see Butcher and Picker, Science 272:60-66 (1996); Springer, Cell 76:301-314 (1994)). In addition, the endothelium present in kidney glomeruli contains a binding protein for the atrial natriuretic peptide (see Lewicki and Protter, in "Hypertension, Pathophysiology, Diagnosis and Management" 2d ed (Raven Press 1995), chapter 61). Thus, endothelial cell markers provide a potential target for directing, for example, a drug to a particular organ in a subject.

In some cases, the metastasis of cancer cells to specific organs also can be due to recognition by the tumor cell of an organ specific marker, including organ specific endothelial cell markers (Fidler and Hart, Science 217:998-1003 (1982)). The pattern of metastasis of many cancers can be explained by assuming that circulating tumor cells are preferentially trapped in the first vascular bed encountered. Thus, the lungs and the liver are the most frequent sites of cancer metastasis. However, some cancers show patterns of metastasis that are not explained by circulatory routing. Metastasis of such cancers can be due, instead, to the presence of selectively expressed address molecules such as endothelial cell surface molecules expressed in the organ to which the cancer metastasizes (see Goetz et al., Intl. J. Canc. 65:192-199 (1996); Zhu et al., Proc. Natl. Acad. Sci., USA 88:9568-9572 (1991); Pauli et al., Canc. Metast. Rev. 9:175-189 (1990); Nicolson, Biochim. Biophys. Acta 948:175-224 (1988)). The identification of molecules that bind to such organ-specific endothelial cell markers can provide a means to prevent tumor cell metastasis to the particular organ.

Breast tumor was selected as target organ or tissue to identify phage expressing peptides that selectively home to this selected organ or tissue because the blood vessels in the organ have unique characteristics. In addition, the vasculature within a tumor generally undergoes active angiogenesis, resulting in the continual formation of new blood vessels to support the growing tumor. Such angiogenic blood vessels are distinguishable from mature vasculature in that angiogenic vasculature expresses unique endothelial cell surface markers, including the αᵥβ₃ integrin (Brooks, Cell 79:1157-1164 (1994)) and receptors for angiogenic growth factors (Mustonen and Alitalo, J. Cell Biol. 129:895-898 (1995); Lappi, Semin. Canc. Biol. 6, 279-288 (1995)). Moreover, tumor vasculature is histologically distinguishable from blood vessel in general in that tumor vasculature is fenestrated (Folkman, Nat. Med. 1:27-31 (1995); Rak et al., Anticancer Drugs 6:3-18 (1995)). Thus, the unique characteristics of tumor vasculature make it a particularly attractive target for determining whether a molecule that homes specifically to a tumor can be identified by *in vivo* panning. Such a tumor homing molecule can be useful for directing an agent such as a chemotherapeutic drug to a tumor, while reducing the likelihood the agent will have a toxic effect on normal, healthy organs or tissues. Moreover, a molecule that homes selectively to tumor vasculature also may have use in targeting other types of neovasculature such as that present in inflammatory, regenerating or wounded tissues.

Using *in vivo* panning, phage expressing various peptides that selectively homed to a breast tumor were identified (see Table 3). Due to the large size of the phage (300 nm) and the short time the phage were allowed to circulate, it is unlikely that a substantial number of phage would have exited the circulatory system. Thus, the organ homing molecules that were identified likely homed to and bound primarily endothelial cell surface markers that are expressed in an organ-specific manner (see, for example, Springer, Cell 76:301-314 (1994)). These results indicate that *in vivo* panning can be used to identify and analyze endothelial cell specificities.

Although the conditions under which the *in vivo* pannings were performed identified organ homing peptides that generally bind to endothelial cell markers, specific binding of phage expressing tumor homing peptides also was observed in tumor parenchyma, indicating that target molecules expressed on specific cells within an organ or tissue also can be identified (see Example III). These results indicate that phage expressing peptides can pass through or leak from the blood vessels in the tumor, possibly due to the fenestrated nature of the blood vessels. Various normal organs such as liver also contain vasculature that allows, for example, phage expressing peptides to contact certain parenchymal cells. Thus, *in vivo* panning can be useful for identifying molecules that home to particular cells or tissue types in an organ as well as to endothelial cells.

Phage peptide display libraries were constructed essentially as described Smith and Scott (*supra,* 1993; see, also, Koivunen et al., Biotechnology 13 : 265 - 270 (1995)). Oligonucleotides encoding peptides having substantially random amino acid sequences were synthesized based on an "NNK" codon, wherein "N" is A, T, C or G and "K" is G or T. "NNK" encodes 32 triplets, which encode the twenty amino acids and an amber STOP codon (Scott and Smith, *supra,* 1990). At least one codon encoding cysteine also was included in each oligonucleotide so that cyclic peptides could be formed through disulfide linkages (see Example I). The oligonucleotides were inserted in frame with the sequence encoding the gene III protein (gIII) in the vector fuse 5 such that a peptide-gIII fusion protein can be expressed. Following expression, the fusion protein is expressed on the surface of the phage containing the vector (Smith and Scott, *supra,* 1993; Koivunen et al., *supra,* 1994b).

Remarkably, following *in vivo* panning, the phage that selectively homed to tumors displayed only a few different peptide sequences (see Table 3 and Example III). In some cases, peptides having the same amino acid sequence were encoded by phage having different oligonucleotide sequences encoding the peptide. These results demonstrate that it is the peptide displayed by the phage, rather than some incidental mutant property of the phage, that directs homing to the selected organ or tissue.

The sequences of the tumor homing motifs do not reveal any significant similarities with known ligands for endothelial cell receptors nor do they resemble any sequences listed in various data banks.

*In vivo* panning was used to identify molecules that selectively home to a tumor. Mice bearing a breast tumor were injected with a phage library and phage that homed to the breast tumor were recovered and the peptides for 14 phage were determined (see Example III.A.). The 14 phage expressed four different peptides, including the peptide CGRECPRLCQSSC (SEQ ID NO: 48), which was expressed by six of the 14 phage.

The identification of breast tumor homing peptides that contain potential integrin binding motifs such as "RE" and "NGR" indicates that *in vivo* panning can be useful to identify integrin binding peptides (see Koivunen et al., *supra,* 1995). The αᵥβ₃ integrin, for example, is expressed in blood vessels undergoing angiogenesis but not in quiescent endothelial cells. Binding of a peptide or antibody antagonist to the αᵥβ₃ integrin induces apoptosis of the angiogenic vessels, possibly by interrupting a signal transduced into the proliferating endothelial cells as a result of αᵥβ₃ integrin binding to the extracellular matrix (Brooks et al., *supra,* 1994).

Since the αᵥβ₃ integrin is expressed by endothelial cells in angiogenic vasculature, experiments were performed to determine whether tumor vasculature that is undergoing angiogenesis can be targeted *in vivo* using methods as disclosed herein. Phage expressing the peptide, CDCRGDCFC (SEQ ID NO: 57; "RGD phage;" see, Koivunen et al., *supra,* 1995), which is known to bind to the αᵥβ₃ integrin, were injected into mice bearing tumors formed from human breast carcinoma cells, human melanoma cells or mouse melanoma cells (see Example IV). The RGD phage selectively homed to each of the tumors, whereas such homing did not occur with control phage. For example, in mice bearing tumors formed by implantation of human breast carcinoma cells, a twenty- to eighty-fold greater number of the RGD phage, as compared to unselected control phage, accumulated in the tumor.

Tissue staining for the phage showed accumulation of the RGD phage in the blood vessels within the tumor, whereas no staining was observed in brain or kidney (control organs). Specificity of tumor homing of the RGD phage was demonstrated by competition experiments, in which coinjection of the RGD-containing peptide greatly reduced tumor homing of the RGD phage, whereas coinjection of a non-RGD-containing control peptide had no effect on homing of the RGD phage (see Example IV). These results demonstrate that the α_{V}β₃ target molecule is expressed on the luminal surface of endothelial cells in a tumor and that a peptide that binds to an α_{V}-containing integrin can bind selectively to this integrin and, therefore, to vasculature undergoing angiogenesis. In view of these results, the skilled artisan would recognize that an RGD peptide or an antibody that binds an α_{V} integrin, such as the monoclonal antibody LM609, which binds an α_{V}β₃ integrin (see Brooks et al., *supra,* 1994), can be used to target an organ or tissue containing angiogenic vasculature, including a tumor containing angiogenic vasculature.

The ability of a molecule that homes to a particular tumor to selectively home to another tumor of the same or a similar histologic type can be determined using, for example, human tumors grown in nude mice or mouse tumors grown in syngeneic mice for these experiments. For example, various human breast cancer cell lines, including MDA-MB-435 breast carcinoma (Price et al., Cancer Res. 50:717-721 (1990)), SKBR-1-II and SK-BR-3 (Fogh et al., J. Natl. Cancer Inst. 59:221-226 (1975)), and mouse mammary tumor lines, including EMT6 (Rosen et al., Intl. J. Cancer 57:706-714 (1994)) and C3-L5 (Lala and Parhar, Intl. J. Cancer 54:677-684 (1993)), are readily available and commonly used as models for human breast cancer. Using such breast tumor models, for example, information relating to the specificity of an identified breast tumor homing molecule for diverse breast tumors can be obtained and molecules that home to a broad range of different breast tumors or provide the most favorable specificity profiles can be identified. In addition, such analyses can yield new information, for example, about tumor stroma, since stromal cell gene expression, like that of endothelial cells, can be modified by the tumor in ways that cannot be reproduced *in vitro.*

Selective homing of a molecule such as a peptide or protein to a selected organ or tissue can be due to specific recognition by the peptide of a particular cell target molecule such as a cell surface receptor present on a cell in the organ or tissue. Selectivity of homing is dependent on the particular target molecule being expressed on only one or a few different cell types, such that the molecule homes to only one or a few organs or tissues. As discussed above, the identified tumor homing peptides at least in part, likely are recognizing endothelial cell surface markers in the blood vessels present in these organs or tissues. However, most different cell types, particularly cell types that are unique to an organ or tissue, can express unique target molecules. Thus, in organs such as liver, spleen or lymph node, where blood circulates through sinusoids formed by the cells specific for the organ, *in vivo* panning can be useful for identifying molecules that home to the particular organ.

The peptides of the invention can be used to direct a moiety to a breast tumor by linking the moiety to the molecule. As used herein, the term "moiety" is used broadly to mean a physical, chemical, or biological material that is linked to an organ homing molecule. For example, a moiety can be an agent such as a drug or can be a chambered microdevice, which can contain an agent. In addition, a moiety can be a molecule such as a protein or nucleic acid, to which an organ homing molecule is grafted for the purpose of directing the protein or nucleic acid to a selected organ or tissue. For example, a peptide organ homing molecule can be expressed as a fusion protein with a desired protein such that the peptide directs the protein to a selected organ.

A moiety can be a detectable label such a radiolabel or can be a cytotoxic agent, including a toxin such as ricin or a drug such as a chemotherapeutic agent or can be a physical, chemical or biological material such as a liposome, microcapsule, micropump or other chambered microdevice, which can be used, for example, as a drug delivery system. Generally, such microdevices, should be nontoxic and, if desired, biodegradable. Various moieties, including microcapsules, which can contain an agent, and methods for linking a moiety or chambered microdevice to a molecule of the invention are well known in the art and commercially available (see, for example, "Remington's Pharmaceutical Sciences" 18th ed. (Mack Publishing Co. 1990), chapters 89-91; Harlow and Lane, Antibodies: A laboratory manual (Cold Spring Harbor Laboratory Press 1988); see, also, Hermanson, *supra,* 1996).

Linking of a moiety to an organ homing molecule for the purpose of directing homing of the moiety to the selected organ or tissue is exemplified by the linking of a brain homing peptide to a RBC, wherein the peptide directed homing of the RBC to brain (see Example II.D.). These results indicate that an organ homing molecule of the invention can be linked to other cell types or to a physical, chemical or biological delivery system such as a liposome or other encapsulating device, which can contain an agent such as drug, in order to direct the cell type or the delivery system to a selected organ or tissue. For example, a tumor homing molecule identified by *in vivo* panning can be linked to a white blood cell (WBC) such as a cytotoxic T cell or a killer cell, wherein upon administration of the tumor homing molecule/WBC complex, the molecule directs homing of the WBC to the tumor, where the WBC can exert its effector function. Similarly, an organ homing molecule can be linked to a liposome or to a microcapsule comprising, for example, a permeable or semipermeable membrane, wherein an agent such as a drug to be delivered to a selected organ or tissue is contained within the liposome or microcapsule.

In one embodiment, an organ homing molecule is linked to a moiety that is detectable external to the subject in order to perform an *in vivo* diagnostic imaging study. For example, *in vivo* imaging using a detectably labeled brain homing peptide can identify a region in the brain where circulation is occluded. For such studies, a gamma ray emitting radionuclide such as indium-111 or technitium-99 can be linked to a brain homing molecule and, following administration to a subject, can be detected using a solid scintillation detector. Alternatively, a positron emitting radionuclide such as carbon-11 or a paramagnetic spin label such as carbon-13 can be linked to the molecule and, following administration to a subject, the localization of the moiety/molecule can be detected using positron emission transaxial tomography or magnetic resonance imaging, respectively.

Such *in vivo* imaging methods also can be used to identify the presence of cancer in a subject by linking an appropriate moiety to a tumor homing molecule, which can recognize a unique target expressed by the tumor cells or by the blood vessels formed by angiogenesis in the tumor. Such methods can identify a primary tumor as well as a metastatic lesion, which may not be detectable using other methods. Having identified the presence of such cancer, in another embodiment of the invention, the tumor homing molecule can be linked to a cytotoxic agent such as ricin or a cancer chemotherapeutic agent in order to direct the moiety to the tumor or can be linked to a chambered microdevice, which can contain, for example, a chemotherapeutic drug or other cytotoxic agent. Such a method can allow selective killing of the tumor, while substantially sparing normal tissues.

When administered to a subject, the molecule/moiety complex is administered as a pharmaceutical composition containing, for example, the complex and a pharmaceutically acceptable carrier. Pharmaceutically acceptable carriers are well known in the art and include, for example, aqueous solutions such as water or physiologically buffered saline or other solvents or vehicles such as glycols, glycerol, oils such as olive oil or injectable organic esters.

A pharmaceutically acceptable carrier can contain physiologically acceptable compounds that act, for example, to stabilize or to increase the absorption of the complex. Such physiologically acceptable compounds include, for example, carbohydrates, such as glucose, sucrose or dextrans, antioxidants, such as ascorbic acid or glutathione, chelating agents, low molecular weight proteins or other stabilizers or excipients. One skilled in the art would know that the choice of a pharmaceutically acceptable carrier, including a physiologically acceptable compound, depends, for example, on the route of administration of the composition. The pharmaceutical composition also can contain an agent such as a cancer therapeutic agent.

One skilled in the art would know that a pharmaceutical composition containing an organ homing molecule can be administered to a subject by various routes including, for example, orally or parenterally, such as intravenously. The composition can be administered by injection or by intubation. The pharmaceutical composition also can be an organ homing molecule linked to liposomes or other polymer matrices, which can have incorporated therein, for example, a drug such as a chemotherapeutic agent (Gregoriadis, Liposome Technology, Vol. 1 (CRC Press, Boca Raton, FL 1984). Liposomes, for example, which consist of phospholipids or other lipids, are nontoxic, physiologically acceptable and metabolizable carriers that are relatively simple to make and administer.

For the diagnostic or therapeutic methods disclosed herein, an effective amount of the molecule/moiety complex must be administered to the subject. As used herein, the term "effective amount" means the amount of the complex that produces the desired effect. An effective amount often will depend on the moiety linked to the organ homing molecule. Thus, a lesser amount of a radiolabeled molecule can be required for imaging as compared to the amount of a drug/molecule complex administered for therapeutic purposes. An effective amount of a particular molecule/moiety for a specific purpose can be determined using methods well known to those in the art.

The route of administration of an organ homing molecule will depend, in part, on the chemical structure of the molecule. Peptides, for example, are not particularly useful when administered orally because they can be degraded in the digestive tract. However, methods for chemically modifying peptides to render them less susceptible to degradation by endogenous proteases or more absorbable through the alimentary tract are well known (see, for example, Blondelle et al., *supra,* 1995; Ecker and Crooke, *supra,* 1995; Goodman and Ro, *supra,* 1995). Such methods can be performed on peptides identified by *in vivo* panning. In addition, methods for preparing libraries of peptide analogs such as peptides containing D-amino acids; peptidomimetics consisting of organic molecules that mimic the structure of peptide; or peptoids such as vinylogous peptoids, are known in the art and can be used to identify molecules that home to a selected organ or tissue and are stable for oral administration.

Organ homing molecules obtained using the methods disclosed herein also can be useful for identifying the presence of a target molecule such as a cell surface receptor or a ligand for a receptor, which is recognized by the organ homing peptide, or for substantially isolating the target molecule. For example, an organ homing peptide can be linked to a solid support such as a chromatography matrix. The linked peptide then can be used for affinity chromatography by passing an appropriately processed sample of a selected organ or tissue over the column in order to bind a particular target molecule. The target molecule, which forms a complex with the organ homing molecule, then can be eluted from the column and collected in a substantially isolated form. The substantially isolated target molecule can be characterized using well known methods. An organ homing molecule also can be linked to a detectable moiety such as a radionuclide, a fluorescent molecule, an enzyme or biotin and can be used, for example, to screen a sample in order to detect the presence of the target molecule or to follow the target molecule during various isolation steps.

The disclosed methods were used to identify peptides that selectively home to a selected organ or tissue. Thus, the present invention provides organ homing peptides, namely the breast tumor homing peptides CGRECPRLCQSSC (SEQ ID NO: 48), CGEACGGQCALPC (SEQ ID NO: 49) and CNGRCVSGCAGRC (SEQ ID NO: 50).

It should be recognized that cysteine residues were included in the peptides such that cyclization of the peptides could be effected. In fact, the peptides containing at least two cysteine residues cyclize spontaneously. However, such cyclic peptides also can be active when present in a linear form (see, for example, Koivunen et al., J. Biol. Chem. 268:20205-20210 (1993)). Thus, in some cases one or both of the cysteine residues in a peptide can be deleted without significantly affecting the organ homing activity of a peptide of the invention. Similarly, the amino acid residues N-terminal and C-terminal to the first and last cysteine residues, respectively, in a peptide can be dispensable without substantially altering homing activity of the peptide. Methods for determining the necessity of a cysteine residue or of amino acid residues N-terminal or C-terminal to a cysteine residue for organ homing activity of a peptide of the invention are routine and well known in the art.

An organ homing peptide is useful, for example, for targeting a desired moiety to the selected organ or tissue as discussed above. In addition, an organ homing peptide can be used to identify the presence of a target molecule in a sample. As used herein, the term "sample" is used in its broadest sense to mean a cell, tissue, organ or portion thereof that is isolated from the body. A sample can be, for example, a histologic section or a specimen obtained by biopsy or cells that are placed in or adapted to tissue culture. If desired, a sample can be processed, for example, by homogenization, which can be an initial step for isolating the target molecule to which an organ homing molecule binds.

An organ homing peptide can be used to identify the target molecule.

The disclosed *in vivo* panning method can be used to detect four different kinds of target molecules in tumors. First, because tumor vasculature undergoes active angiogenesis, target molecules that are characteristic of angiogenic vasculature, in general, or angiogenic tumor vasculature, in particular, can be identified. Second, vascular target molecules that are characteristic of the tissue of origin of the tumor can be identified. Third, target molecules that are expressed in the vasculature of a particular type of tumor can be identified. Fourth, tumor stroma or tumor cell target molecules can be identified due to the fenestrated nature of tumor vasculature, which allows the potential organ homing molecules to leave the circulation and contact the tumor parenchyma.

As an alternative to using an organ or tissue sample, extracts of cultured endothelial cells can be used as the starting material in order to enhance the concentration of the receptor in the sample. The presence of the receptor can be established by using phage binding and cell attachment assays (see, for example, Barry et al., Nat. Med. 2:299-305 (1996).

A cell line expressing a particular receptor can be identified and surface iodination of the cells can be used to label the receptor. The cells then can be extracted with octylglucoside and the extract can be fractionated by affinity chromatography using a brain homing peptide (see Table 1) coupled to a matrix such as Sepharose^{™}. Extracts prepared from HUVEC cells (human umbilical vein endothelial cells) can be used as a control for the brain-derived endothelial cells. The purified receptor can be microsequenced and antibodies can be prepared. If desired, oligonucleotide probes can be prepared and used to isolate cDNA clones encoding the receptor. Alternatively, an anti-receptor antibody can be used to isolate a cDNA clone from an expression library (see Argraves et al., J. Cell Biol. 105:1183-1190 (1987), which is incorporated herein by reference).

As an alternative to isolating the receptor, a nucleic acid encoding the receptor can be isolated using a mammalian cell expression cloning system such as the COS cell system. An appropriate library can be prepared, for example, using mRNA from primary brain endothelial cells (Lathey et al., Virology 176:266-273 (1990)). The nucleic acids can be cloned into the pcDNAI vector (Invitrogen), for example. Cells expressing a cDNA for the receptor can be selected by binding to the peptide. Purified phage can be used as the carrier of the peptide and can be attached to magnetic beads coated, for example, with anti-M13 antibodies (Pharmacia). Cells that bind to the peptide coating can be recovered using a magnet and the plasmids can be isolated. The recovered plasmid preparations then can be divided into pools and examined in COS cell transfections. The procedure can be repeated until single plasmids are obtained that can confer upon the COS cells the ability to bind the peptide.

The following examples are intended to illustrate but not limit the present invention.

### EXAMPLE I

### IN VIVO PANNING (technical background)

This example demonstrates methods for preparing a phage library and screening the library using *in vivo* panning to identify phage expressing peptides that home to a selected organ or tissue.

### A. Preparation of phage libraries:

Phage display libraries were constructed using the fuse 5 vector as described by Koivunen et al. (*supra,* 1995; see, also, Koivunen et al. Meth. Enzymol. 245:346-369 (1994b), which is incorporated herein by reference). Six libraries encoding peptides designated CX₅C (SEQ ID NO: 36), CX₆C (SEQ ID NO: 37), CX₇C (SEQ ID NO: 38), CX₉ (SEQ ID NO: 39), X₂CX₁₄CX₂ (SEQ ID NO: 40) and X₂CX₁₈ (SEQ ID NO: 41) were prepared, where "C" indicates cysteine and "X_{N}" indicates the given number of individually selected amino acids. These libraries can display cyclic peptides when at least two cysteine residues are present in the peptide.

Oligonucleotides were constructed such that "C" was encoded by the codon TGT and "X_{N}" was encoded by NNK, where "N" is equal molar mixtures of A, C, G and T, and where "K" is equal molar mixtures of G and T. Thus, the peptide represented by CX₅C (SEQ ID NO: 36) can be represented by an oligonucleotide having the sequence TGT(NNK)₅TGT (SEQ ID NO: 42). Oligonucleotides were made double stranded by 3 cycles of PCR amplification, purified and ligated to the nucleic acid encoding the gene III protein in the fuse 5 vector such that, upon expression, the peptide is present as a fusion protein at the N-terminus of the gene III protein.

The vectors were transfected by electroporation into MC1061 cells. Bacteria were cultured for 24 hr in the presence of 20 µg/ml tetracycline, then phage were collected from the supernatant by precipitation twice using polyethylene glycol. Each library contained about 5 x 10⁹ to 5 x 10¹⁴ transducing units (TU; individual recombinant phage).

### B. In vivo panning of phage:

A mixture of phage libraries containing 1 x 10¹⁴ TU was diluted in 200 µl DMEM and injected into the tail vein of anesthetized BALB\c mice (2 month old females; Jackson Laboratories; Bar Harbor ME); Avertin^{™} (0.015 ml/g) was used as anesthetic (see Pasqualini and Ruoslahti, *supra,* 1996). After 1-4 minutes, mice were snap frozen in liquid nitrogen. To recover the phage, carcasses were partially thawed at room temperature for 1 hr, organs were collected and weighed, then were ground in 1 ml DMEM-PI (DMEM containing protease inhibitors (PI); phenylmethylsulfonyl fluoride (PMSF; 1 mM), aprotinin (20 µg/ml), leupeptin (1 µg/ml)).

Organ samples were washed 3 times with ice cold DMEM-PI containing 1% bovine serum albumin (BSA), then directly incubated with 1 ml K91-kan bacteria for 1 hr. Ten ml NZY medium containing 0.2 µg/ml tetracycline (NZY/tet) was added to the bacterial culture, the mixture was incubated in a 37°C shaker for 1 hr, then 200 µl aliquots were plated in agar plates containing 40 µg/ml tetracycline (tet/agar).

Individual colonies containing phage recovered from brain or kidney were grown for 16 hr in 5 ml NZY/tet. The bacterial cultures obtained from the individual colonies were pooled and the phage were purified and re-injected into mice as described above for a second round of *in vivo* panning. A third round of panning also was performed. Phage DNA was purified from individual bacterial colonies obtained from the second and the third round of *in vivo* panning and the DNA sequences encoding the peptides expressed by selected phage were determined (see Koivunen et al., *supra,* 1994b).

### EXAMPLE II

### CHARACTERIZATION OF PEPTIDES THAT HOME TO A SELECTED ORGAN

This example demonstrates that an organ homing peptide of the invention selectively homes to a selected organ and that an organ homing peptide identified by *in vivo* panning can be used to direct a moiety to a selected organ.

### A. Brain is the selected organ (technical background)

Three rounds of *in vivo* panning in mice were performed. Kidney was used as a control organ. Mice were injected with two different mixtures of phage libraries. The first mixture contained libraries encoding CX₉ (SEQ ID NO: 39), CX₅C (SEQ ID NO: 36), CX₆C (SEQ ID NO: 37) and CX₇C (SEQ ID NO: 38) peptides (CX₅₋₇/CX₉ mixture; SEQ ID NOS: 36-39). The second mixture contained libraries encoding X₂CX₁₄CX₂ (SEQ ID NO: 40) and X₂CX₁₈ (SEQ ID NO: 41) peptides (X₂CX₁₈/X₂CX₁₄CX₂ mixture; SEQ ID NOS: 40 and 41).

The phage library mixtures were administered to mice via tail vein injection. Phage input was 1 x 10¹⁶ TU of the CX₅₋₇/CX₉ (SEQ ID NOS: 36-39) mixture or 1 x 10¹⁴ TU of the X₂CX₁₈/X₂CX₁₄CX₂ (SEQ ID NOS: 40 and 41) mixture. Phage were recovered from the brains of the injected mice, the recovered phage were amplified *in vitro,* then a second and a third round of *in vivo* panning was performed. During the second and third rounds of panning, phage were recovered from brain and from kidney and the number of TU from each organ was compared. This comparison revealed that 6x more phage from the CX₅₋₇/CX₉ (SEQ ID NOS: 36-39) mixture bound to brain than to kidney in the second round and 13x more of the CX₅₋₇/CX₉ (SEQ ID NOS: 36-39) phage bound to brain than to kidney in the third round of panning. Following administration of the X₂CX₁₈/X₂CX₁₄CX₂ (SEQ ID NOS: 40 and 41) mixture, an 11x and 8x enrichment of phage homing to the brain as compared to kidney occurred during the second and third rounds of panning, respectively. Thus, substantial enrichment of phage binding to the brain was observed following the second and third rounds of *in vivo* panning.

The amino acid sequences were determined for the inserts present in 73 cloned phage that were recovered from brain during the second and third rounds of *in vivo* panning. Peptides containing an SRL motif predominated (36% of the clones sequenced; see SEQ ID NOS: 1, 3 and 5), followed by peptides containing the VLR motif (20.5% of the clones; see SEQ ID NOS: 4 and 16) and the peptide CENWWGDVC (SEQ ID NO: 2; 19% of the clones; see Table 1). Other peptides that,occurred much less frequently, but were present more than once, included CGVRLGC (SEQ ID NO: 6), CKDWGRIC (SEQ ID NO: 7), CLDWGRIC (SEQ ID NO: 8) and CTRITESC (SEQ ID NO: 9). Eight other sequences appeared only one time each and were not characterized further. The SRL tripeptide motif was present in several different sequence contexts, indicating that the nucleic acids encoding the peptides were derived from a number of independent phage. These results indicate that the selection of the peptides containing the SRL motif represents the specific binding of several independent phage displaying peptides with the SRL sequence and is not an artifact due, for example, to phage amplification. In addition, in some cases, different phage expressed peptides that had the same amino acid sequence, but were encoded by oligonucleotides having different sequences, thus confirming that homing of a particular phage to an organ is due to the specific peptide expressed on the phage.

To determine the specificity of brain homing of the individual motifs identified, phage displaying the predominant motifs were amplified individually, diluted to the same input titer and administered to mice. Following administration, brain and kidney were removed and the number of TU of phage in each organ was determined. The enrichment ratio of phage recovered from the selected organ, brain, as compared to the control organ, kidney, revealed that phage displaying one of the four most recovered peptides, CNSRLHLRC (SEQ ID NO: 1), CENWWGDVC (SEQ ID NO: 2), WRCVLREGPAGGCAWFNRHRL (SEQ ID NO: 16), CLSSRLDAC (SEQ ID NO: 3), each selectively targeted the brain as compared to the kidney. Specifically, the ratio of selective homing (brain:kidney) was about 8 for CNSRLHLRC (SEQ ID NO: 1) and for CLSSRLDAC (SEQ ID NO: 3), 4 for CENWWGDVC (SEQ ID NO: 2) and 9 for WRCVLREGPAGGCAWFNRHRL (SEQ ID NO: 16).

**TABLE 1**

| PEPTIDES FROM PHAGE RECOVERED FROM BRAIN | |
|---|---|
| | # PHAGE DISPLAYING |
| A.A. SEQUENCE | SAME A.A. SEQUENCE |
| | (% PHAGE) |
| CX₅₋₇C/CX₉ (36-39)* library: | |
| CNSRLHLRC (1)* | 16 (21.9%) |
| CENWWGDVC (2) | 14 (19.2%) |
| CLSSRLDAC (3) | 6 (8.2%) |
| CVLRGGRC (4) | 5 (6.8%) |
| CNSRLQLRC (5) | 4 (5.5%) |
| CGVRLGC (6) | 3 (4.1%) |
| CKDWGRIC (7) | 2 (2.8%) |
| CLDWGRIC (8) | 2 (2.8%) |
| CTRITESC (9) | 2 (2.8%) |
| CETLPAC (10) | 1 (1.4%) |
| CRTGTLFC (11) | 1 (1.4%) |
| CGRSLDAC (12) | 1 (1.4%) |
| CRHWFDVVC (13) | 1 (1.4%) |
| CANAQSHC (14) | 1 (1.4%) |
| CGNPSYRC (15) | 1 (1.4%) |

| X₂CX₁₈/X₂CX₁₄CX₂ (SEO ID NOS: 40 and 41) library: | |
|---|---|
| WRCVLREGPAGGCAWFNRHRL (16) | 10 (13.7%) |
| YPCGGEAVAGVSSVRTMCSE (17) | 1 (1.4%) |
| LNCDYQGTNPATSVSVPCTV (18) | 1 (1.4%) |

| | |
|---|---|
| * - numbers in parentheses indicate SEQ ID NO:. | |

Two control phage showed low binding to both brain and kidney. These results demonstrate that *in vivo* panning can be used to screen phage display libraries in order to identify phage expressing peptides that home to a selected organ.

### B. Kidney as the selected organ (technical background)

The same methodology used to isolate phage expressing brain homing peptides was used to isolate phage expressing peptides that home to kidney. In these experiments, brain was used as the control organ. A mixture of the CX₅C (SEQ ID NO: 36) and the CX₆C (SEQ ID NO: 37) libraries was administered as described above. Homing of phage to the kidney was obtained and an approximately 3x to 7x enrichment of phage homing to kidney was observed following a second round of *in vivo* panning.

The amino acid sequences were determined for the inserts in 48 cloned phage that homed to kidney. The peptides expressed by these phage were represented by two predominant sequences, CLPVASC (SEQ ID NO: 21; 46% of the clones sequenced) and CGAREMC (SEQ ID NO: 22; 17% of the clones; see Table 2). In addition, the peptide CKGRSSAC (SEQ ID NO: 23) appeared three times and three other peptides were present twice each. Phage expressing a CLPVASC (SEQ ID NO: 21), CGAREMC (SEQ ID NO: 22) or CKGRSSAC (SEQ ID NO: 23) peptide each exhibited selective homing to kidney; the ratio of selective kidney:brain homing was 7 for CLPVASC (SEQ ID NO: 21), 3 for CGAREMC (SEQ ID NO: 22) and 2 for CKGRSSAC (SEQ ID NO: 23).

These results demonstrate that the *in vivo* panning method is a generally applicable method for screening a phage library to identify phage expressing peptides that home to a selected organ. Database

**TABLE 2**

| PEPTIDES FROM PHAGE RECOVERED FROM KIDNEY | |
|---|---|
| | # PHAGE DISPLAYING |
| A.A. SEQUENCE | SAME A.A. SEQUENCE |
| | (% PHAGE) |
| CLPVASC (21)* | 22 (45.8%) |
| CGAREMC (22) | 8 (16.7%) |
| CKGRSSAC (23) | 3 (6.2%) |
| CWARAQGC (24) | 2 (4.2%) |
| CLGRSSVC (25) | 2 (4.2%) |
| CTSPGGSC (26) | 2 (4.2%) |
| CMGRWRLC (27) | 1 (2.1%) |
| CVGECGGC (28) | 1 (2.1%) |
| CVAWLNC (29) | 1 (2.1%) |
| CRRFQDC (30) | 1 (2.1%) |
| CLMGVHC (31) | 1 (2.1%) |
| CKLLSGVC (32) | 1 (2.1%) |
| CFVGHDLC (33) | 1 (2.1%) |
| CRCLNVC (34) | 1 (2.1%) |
| CKLMGEC (35) | 1 (2.1%) |

| | |
|---|---|
| * - numbers in parentheses indicate SEQ ID NO:. | |

searches did not reveal any significant homology of the brain homing or kidney homing peptides to known ligands for endothelial cell receptors.

### C. Peptide homing is specific:

In order to confirm the specificity of a peptide for directing homing to a selected organ, peptide competition experiments were performed. The cyclic peptide, CLSSRLDAC (SEQ ID NO: 3), which is one of the brain homing peptides (see Table 1) was synthesized' (Immunodynamics; La Jolla CA) and purified by HPLC. The effect on the homing of phage expressing CLSSRLDAC (SEQ ID NO: 3), CENWWGDVC (SEQ ID NO: 2) or WRCVLREGPAGGCAWFNRHRL (SEQ ID NO: 16) to the brain was examined in order to determine whether co-administration of the synthetic peptide affected homing of the phage.

Phage expressing CLSSRLDAC (SEQ ID NO: 3), CENWWGDVC (SEQ ID NO: 2) or WRCVLREGPAGGCAWFNRHRL (SEQ ID NO: 16) were titrated to the same concentration and 1 x 10⁸ TU was injected into mice alone, or with 100 µg purified synthetic cyclic CLSSRLDAC (SEQ ID NO: 3) peptide. The synthetic CLSSRLDAC (SEQ ID NO: 3) peptide-inhibited the homing of phage expressing CLSSRLDAC (SEQ ID NO: 3) by about 60%. This result demonstrates that the homing of the phage to brain is specifically due to the expression on the phage of the CLSSRLDAC (SEQ ID NO: 3) peptide.

The synthetic CLSSRLDAC (SEQ ID NO: 3) peptide also inhibited homing of phage expressing the WRCVLREGPAGGCAWFNRHRL (SEQ ID NO: 16) peptide by about 60%, but did not affect homing of CENWWGDVC (SEQ ID NO: 2) phage. This result indicates that the CLSSRLDAC (SEQ ID NO: 3) and WRCVLREGPAGGCAWFNRHRL (SEQ ID NO: 16) peptides can recognize the same target molecule in brain, whereas the CENWWGDVC (SEQ ID NO: 2) peptide recognizes a different target.

### D. The brain homing peptide CLSSRLDAC (SEO ID NO: 3) directs red blood cells to brain (technical background)

The synthetic cyclic CLSSRLDAC (SEQ ID NO: 3) peptide (1 mg) was labeled with iodine-125 using the Bolton and Hunter reagent (Amersham; Arlington Heights IL). Labeled peptide was purified by reverse phase HPLC on Sep-Pak^{™} cartridges (Waters, Millipore Corp.; Milford PA) and the ¹²⁵I-peptide (200 µg) was coupled to 1 ml glutaraldehyde-stabilized sheep RBC (Sigma Chemical Co.; St. Louis MO) according to the manufacturer's instructions.

Fifty µl ¹²⁵I-peptide/RBC (200,000 cpm), alone or with 10 mM of unlabeled CLSSRLDAC (SEQ ID NO: 3), which is a brain homing peptide, or CVRLNSLAC (SEQ ID NO: 43), which has no brain homing activity, was injected into the tail vein. After 2 min, each mouse was perfused through the heart with 50 ml DMEM and the brain and kidney were removed and assayed for radioactivity.

Approximately twice as much of the CLSSRLDAC/RBC complex (SEQ ID NO: 3) homed to brain than to kidney. Coadministration of unlabeled CLSSRLDAC (SEQ ID NO: 3) with the complex essentially completely inhibited brain homing of the complex but had no effect on complex localizing to the kidney, indicating that localization of the complex in the kidney was non-specific. Coadministration of unlabeled CVRLNSLAC (SEQ ID NO: 43) had no effect on selective homing of the complex to brain and no effect on the non-specific localization of the complex in the kidney. These results demonstrate that an organ homing peptide identified using *in vivo* panning can be linked to a moiety such as a blood cell and selectively directs homing of the linked moiety to the selected organ.

### E. In vivo panning for molecules that selectively home to an organ containing a component of the reticuloendothelial system

This example demonstrates that enhanced uptake of peptide-displaying phage by the RES in liver and spleen can be blocked by coadministration of non-infective phage, thereby allowing identification of molecules that selectively home to an RES-containing organ.

Approximately 1 x 10⁸ TU of amplified phage expressing a CX₉ (SEQ ID NO: 39) library were injected, iv, into mice, either alone or with a 1000-fold excess of non-infective phage, which are not amplifiable. After 2 min, the mice were sacrificed and phage were rescued from liver, lung, kidney, brain and spleen, and the number of phage (TU) per gram (TU/g) of tissue recovered was determined.

Coadministration of non-infective phage with library phage substantially reduced the amount of phage recovered in liver from about 1300 TU/g to about 200 TU/g and, in spleen from about 300 TU/g to about 200 TU/g. In comparison, little, or no difference was observed in the number of phage recovered from brain, kidney or lung when the phage library was administered alone or in combination with the non-infective phage. This result demonstrates that nonspecific uptake of phage by organs such as liver and spleen, which contain an RES component, can be blocked by coadministration of non-infective phage with a phage library.

These results also demonstrated that uptake of phage by the lung was selective. Specifically, recovery of phage from lung essentially identical regardless of whether the phage library was injected alone or was coadministered with non-infective phage. Thus, the high uptake of phage previously observed in the lung (Pasqualini and Ruoslahti, *supra,* 1996) likely is due to the large amount of vasculature in the lungs and is not, for example, due to uptake of the phage by alveolar phagocytes, which constitute an RES component in lung.

In order to demonstrate that the phage that homed to liver when coadministered with non-infective phage, in fact, selectively homed to liver, a phage library was coadministered with non-infective phage, then phage recovered from the liver after a first round of *in vivo* panning were amplified *in vitro* and used for a second round of *in vivo* panning. Phage were recovered from liver and from brain (control organ) and quantitated after each round of *in vivo* panning.

Following the second round of *in vivo* panning, more phage homed to liver (350 TU/g) as compared to brain (200 TU/g). In addition, about 150 TU phage/g liver were recovered after the first round, whereas about 350 TU phage/g liver were recovered after the second round, indicating that the population of phage recovered from the liver following the first round of *in vivo* panning was enriched with phage that selectively home to liver. These results demonstrate that the RES component of an organ such as liver can be blocked, thus allowing use of the *in vivo* panning method to identify a molecule that selectively homes to such an organ.

### EXAMPLE III

### CHARACTERIZATION OF PEPTIDES THAT HOME TO A SELECTED TUMOR TISSUE

This example demonstrates that *in vivo* panning can be used to identify peptides that home to a breast tumor or to a melanoma.

### A. Peptides that home to a human breast carcinoma tumor implanted into a nude mouse

Human 435 breast carcinoma cells (Price et al., Cancer Res. 50:717-721 (1990)) were inoculated into the mammary fat pad of nude mice. When the tumors attained a diameter of about 1 cm, either a phage targeting experiment was performed, in which phage expressing a specific peptide were administered to the tumor bearing mouse, or *in vivo* panning was performed.

The breast tumor bearing mice were injected with 1 x 10⁹ phage expressing a library of CX₃CX₃CX₃C (SEQ ID NO: 47) peptides, where X₃ indicates three groups of independently selected, random amino acids. The phage were allowed to circulate for 4 min, then the mice were anesthetized, snap frozen in liquid nitrogen while under anesthesia, and the tumor was removed. Phage were isolated from the tumor and subjected to two additional rounds of *in vivo* panning.

Following the third round of panning, phage were quantitated and the peptide sequences expressed by 14 different cloned phage were determined. The 14 cloned phage expressed 4 different peptides (Table 3). These results demonstrate that the *in vivo* panning method can identify molecules that selectively home to breast tumor tissue.

### B. Peptides that home to B16 mouse melanoma cells implanted into a mouse (technical background)

The general applicability of the *in vivo* panning method to identify peptides that selectively home to a tumor was examined by performing *in vivo* panning in mice bearing an implanted mouse melanoma tumor.

**TABLE 3**

| PEPTIDES FROM PHAGE RECOVERED FROM HUMAN BREAST CANCER | | |
|---|---|---|
| SEQUENCE | | |
| CX₃CX₃CX₃C (47)* library | # clones | % of total phage sequenced |
| CGRECPRLCQSSC (48) | 6 | 42.9 |
| CGEACGGQCALPC (49) | 4 | 28.6 |
| CNGRCVSGCAGRC (50) | 3 | 21.4 |
| CGLMCQGACFDVC (51) | 1 | 7.1 |

| | | |
|---|---|---|
| * - numbers in parentheses indicate SEQ ID NO:. | | |

Mice bearing a melanoma were produced by implantation of B16B15b mouse melanoma cells, which produce highly vascularized tumors. B16B15b mouse melanoma cells were injected subcutaneously into the mammary fat pad of nude mice (2 months old) and tumors were allowed to grow until the diameter was about 1 cm. *In vivo* panning was performed as disclosed above. Approximately 1 x 10¹² transducing units of phage expressing the CX₅C (SEQ ID NO: 36), CX₆C (SEQ ID NO: 37) or CX₅C (SEQ ID NO: 38) library were injected, iv, and allowed to circulate for 4 min. Mice then were snap frozen in liquid nitrogen while under anesthesia, tumor tissue and brain (control organ) were removed, and phage were isolated as described above. Three rounds of *in vivo* panning were performed.

The amino acid sequences were determined for the inserts in 89 cloned phage recovered from the B16B15b tumors. The peptides expressed by these phage were represented by two predominant sequences, CLSGSLSC (SEQ ID NO: 55; 52% of the clones sequenced) and WGTGLC (SEQ ID NO: 52; 25% of the clones; see Table 4). Reinfection of phage expressing one of the selected peptides resulted in approximately three-fold enrichment of phage homing to the tumor relative to brain.

| **TABLE 4** | | |
|---|---|---|
| PEPTIDES FROM PHAGE RECOVERED FROM MOUSE B16B15b MELANOMA | | |
| SEQUENCE | | |
| CX₅C (36)* library sequenced | # clones | % of total phage |
| WGTGLC (52) | 22 | 25% |
| CSVANSC (53) | 3 | 3% |
| CSSTMRC (54) | 2 | 2% |

| CX₆C (37) library | | |
|---|---|---|
| CLSGSLSC (55) | 46 | 52% |

| CX₇C (38) library | | |
|---|---|---|
| CIEGVLGGC (56) | 2 | 2% |

| | | |
|---|---|---|
| * - numbers in parentheses indicate SEQ ID NO:. | | |

Localization of the phage expressing a tumor homing peptide in the mouse organs was examined by immunohistochemical staining of the tumor and various other tissues. 1 x 10⁹ pfu of a control (insertless) phage or a phage expressing the tumor homing peptide, CLSGSLSC (SEQ ID NO: 50), were injected, iv, into tumor bearing mice and allowed to circulate for 4 min. The mouse then was perfused with DMEM and various organs, including the tumor were removed and fixed in Bouin's solution. Histologic sections were prepared and stained using anti-M13 (phage) antibodies (see Pasqualini and Ruoslahti, *supra*, 1996).

Strong immunostaining was evident in the melanoma obtained from a mouse injected with phage expressing the CLSGSLSC (SEQ ID NO: 50) tumor homing peptide. Staining of the melanoma generally was localized to the blood vessels within the tumor, although some staining also was present in the tumor parenchyma. Essentially no staining was observed in a tumor obtained from a mouse injected with the insertless control phage or in skin or in kidney samples obtained from mice injected with either phage. However, immunostaining was detected in the liver sinusoids and in spleen, indicating that phage can be trapped nonspecifically in organs containing RES.

These results demonstrate that the *in vivo* panning method is a generally applicable method for screening a phage library to identify phage expressing peptides that home to a selected organ or tissue, including a tumor.

### EXAMPLE IV

### IN VIVO TARGETING OF A PHAGE EXPRESSING AN AN RGD PEPTIDE TO A TUMOR

Human 435 breast carcinoma cells were inoculated into the mammary fat pad of nude mice. When the tumors attained a diameter of about 1 cm, phage expressing a specific RGD-containing peptide were administered to the tumor bearing mouse. Similar results to those discussed below also were obtained with nude mice bearing tumors formed by implantation of human melanoma C8161 cells or by implantation of mouse B16 melanoma cells.

1 x 10⁹ phage expressing the RGD-containing peptide, CDCRGDCFC (SEQ ID NO: 57; see, Koivunen et al., *supra,* 1995) or control (insertless) phage were injected intravenously (iv) into the mice and allowed to circulate for 4 min. The mice then were snap frozen while under anesthesia, and various organs, including tumor, brain and kidney, were removed and the phage present in the organs was quantitated (see Pasqualini and Ruoslahti, *supra,* 1996).

Approximately 2-3 times more phage expressing the CDCRGDCFC (SEQ ID NO: 57) peptide were detected in the breast tumor as compared to brain and kidney, indicating the CDCRGDCFC (SEQ ID NO: 57; RGD phage) peptide resulted in selective homing of the phage to the breast tumor. In a parallel study, unselected phage, which express various, diverse peptides, were injected into tumor-bearing mice and various organs were examined for the presence of phage. Far more phage were present in kidney and, to a lesser extent, brain, as compared to the tumor. Thus, the 80-fold more RGD-expressing phage than unselected phage concentrated in the tumor. These results indicate that phage expressing the RGD-containing peptide home to a tumor, possibly due to the expression of the αᵥβ₃ integrin on blood vessels forming in the tumor.

Specificity of the breast tumor homing peptide was demonstrated by competition experiments, in which coinjection of 500 µg free peptide, ACDCRGDCFCG (SEQ ID NO: 58) with the phage expressing the tumor homing peptide reduced the amount of phage in the tumor by about tenfold, whereas coinjection with the inactive control peptide, GRGESP (SEQ ID NO: 59) essentially had no effect. Immunohistochemical staining for the phage that homed to the breast tumors showed accumulation of the RGD phage in the blood vessels within the tumor, whereas no staining was observed in brain or kidney (control organs). These results demonstrate that phage displaying a peptide that can bind to an integrin expressed on angiogenic vasculature can selectively home *in vivo* to an organ or tissue such as a tumor containing such vasculature.

## Claims

1. A molecule that selectively homes to a breast tumor, which is a peptide having an amino acid sequence selected from the group consisting of CGRECPRLCQSSC (SEQ ID NO: 48), CGEACGGQCALPC (SEQ ID NO: 49) and CNGRCVSGCAGRC (SEQ ID NO: 50).

2. An *in vitro* method of directing a moiety to a breast tumor, comprising the steps of:
a. linking the moiety to a molecule of claim 1 to form a complex comprising said moiety and said molecule; and
b. contacting said complex with the breast tumor, wherein said molecule of claim 1 selectively binds to the breast tumor, thereby directing said moiety to said breast tumor.

3. The method of claim 2, wherein said moiety comprises a detectable label.

4. The method of claim 2 or 3, wherein said moiety comprises cytotoxic agent.

5. The method of claims 2 to 4, wherein said moiety is a liposome, microcapsule or micropump.

6. Use of a molecule according to claim 1 for preparing a pharmaceutical composition for directing a moiety to a breast tumor.

7. Use of claim 6, wherein said moiety comprises a detectable label.

8. Use of claims 6 or 7, wherein said moiety comprises a cytotoxic agent.

9. Use of claims 6 to 8, wherein said moiety is a liposome, microcapsule or micropump.

## Patentansprüche

1. Ein Molekül, das sich selektiv in einem Brusttumor einfindet, welches ein Peptid mit einer Aminosäuresequenz ausgewählt aus der Gruppe bestehend aus CGRECPRLCQSSC (SEQ ID NR: 48), CGEACGGQCALPC (SEQ ID NR: 49) und CNGRCVSGCAGRC (SEQ ID NR: 50) ist.

2. Ein *in vitro* Verfahren zum Führen eines Rests zu einem Brusttumor, Schritte umfassend, bei denen man:
a. den Rest mit einem Molekül nach Anspruch 1 verbindet, um einen Komplex zu bilden, welcher den Rest und das Molekül umfasst; und
b. den Komplex mit dem Brusttumor in Kontakt bringt, wobei das Molekül nach Anspruch 1 selektiv an den Brusttumor bindet und **dadurch** den Rest zu dem Brusttumor führt.

3. Verfahren nach Anspruch 2, **dadurch gekennzeichnet, dass** der Rest eine detektierbare Markierung umfasst.

4. Verfahren nach den Ansprüchen 2 oder 3, **dadurch gekennzeichnet, dass** der Rest ein zytotoxisches Agens umfasst.

5. Verfahren nach den Ansprüchen 2 bis 4, **dadurch gekennzeichnet, dass** der Rest ein Liposom, eine Mikrokapsel oder eine Mikropumpe ist.

6. Verwendung eines Moleküls nach Anspruch 1 zur Herstellung einer pharmazeutischen Zusammensetzung zum Führen eines Rests zu einem Brusttumor.

7. Verwendung nach Anspruch 6, **dadurch gekennzeichnet, dass** der Rest eine detektierbare Markierung umfasst.

8. Verwendung nach den Ansprüchen 6 oder 7, **dadurch gekennzeichnet, dass** der Rest ein zytotoxisches Agens umfasst.

9. Verwendung nach den Ansprüchen 6 bis 8, **dadurch gekennzeichnet, dass** der Rest ein Liposom, eine Mikrokapsel oder eine Mikropumpe ist.

## Revendications

1. Molécule d'adressage sélectif vers une tumeur du sein, qui est un peptide ayant une séquence d'acides aminés choisie dans le groupe constitué de CGRECPRLCQSSC (SEQIDNO: 48), CGEACGGQCALPC (SEQIDNO: 49) et CNGRCVSGCAGRC (SEQ ID NO : 50).

2. Procédé *in vitro* pour diriger un fragment vers une tumeur du sein; comprenant les étapes consistant à :
a. lier le fragment à une molécule selon la revendication 1 pour former un complexe comprenant ledit fragment et ladite molécule ; et
b. mettre en contact ledit complexe avec la tumeur du sein, ladite molécule selon la revendication 1 se liant sélectivement à la tumeur du sein, dirigeant ainsi ledit fragment vers ladite tumeur du sein.

3. Procédé selon la revendication 2, dans lequel ledit fragment comprend un marqueur détectable.

4. Procédé selon la revendication 2 ou 3, dans lequel ledit fragment comprend un agent cytotoxique.

5. Procédé selon les revendications 2 à 4, dans lequel ledit fragment est un liposome, une microcapsule ou une micropompe.

6. Utilisation d'une molécule selon la revendication 1 pour préparer une composition pharmaceutique pour diriger un fragment vers une tumeur du sein.

7. Utilisation selon la revendication 6, dans laquelle ledit fragment comprend un marqueur détectable.

8. Utilisation selon les revendications 6 ou 7, dans laquelle ledit fragment comprend un agent cytotoxique.

9. Utilisation selon les revendications 6 à 8, dans laquelle ledit fragment est un liposome, une microcapsule ou une micropompe.
